# EUROPEAN PATENT SPECIFICATION

(11) **EP 1 187 559 B1**
(45) Date of publication and mention of the grant of the patent: **12.09.2007**
(21) Application number: 00943084.4
(22) Date of filing: 22.06.2000
(51) Int. Cl.: A61B 17/064, A61B 17/068

(54) **GERD TREATMENT APPARATUS**
GERD VORRICHTUNG
DISPOSITIF A TRAITEMENT DU REFLUX GASTRO-OESOPHAGIEN PATHOLOGIQUE

(30) Priority: 22.06.1999 US 140492 P; 07.03.2000 US 520273; 07.03.2000 US 519945; 19.05.2000 US 574424
(43) Date of publication of application: 20.03.2002
(60) Divisional application: 04076389.8
(73) Proprietor: NDO Surgical, Inc., Mansfield, MA 02048 (US)
(72) Inventor: LAUFER, Michael, D., Menlo Park, CA 94025 (US); CERIER, Jeffrey, C., Franklin, MA 02038 (US); CRUZ, Amos, G., Franklin, MA 02038 (US)
(74) Representative: Arends, William Gerrit
(86) International application number: PCT/US2000/017260
(87) International publication number: WO 2000/078227

(56) References cited:
- EP-A- 0 593 920
- EP-A- 0 646 356
- WO-A-99/22649
- FR-A- 2 768 324
- US-A- 4 880 015
- US-A- 5 395 030
- US-A- 5 403 326
- US-A- 5 897 562

## Description

### BACKGROUND

This invention relates to an apparatus for reconfiguring tissue, and more particularly to reconfiguring tissue in the vicinity of the gastroesophageal junction.

Gastroesophageal reflux disease (GERD) is a common upper-gastrointestinal disorder in which acidic contents of the stomach flow inappropriately from the stomach into the esophagus. Backflow of gastric contents into the esophagus results when gastric pressure is sufficient to overcome the resistance to flow that normally exists at the gastroesophageal junction (GEJ) or when gravity acting on the contents is sufficient to cause flow through the GEJ. Medication, open surgical procedures, minimally invasive surgical techniques, and endoscopic techniques are known for treating GERD.

There have been some attempts to treat reflux disease at flexible endoscopy. An early endoscopic approach to control GERD was to inject collagen in and around the LES. O'Connor and Lehman treated ten patients by this method with some success, although some patients required further injections at the LES to maintain symptomatic relief. O'Connor KW and Lehman GA 1988, Gastrointest Endosc 34:106-12.
Donahue et al. demonstrated that GERD, induced with high-dose intravenous atropine in dogs, could be controlled by injection of 5 percent morruhate sodium in the proximal gastric region 1 to 2 cm distal to the LES at flexible endoscopy and suggested that the proximal gastric sclerosis caused by the injection formed an effective antireflux barrier. Donahue PE et al. 1990, Gastrointest Endosc 36:253-6; Donahue PE et al. 1992, World J Surg 16:343-6. Endoscopic proximal gastric sclerosis induced by Nd:YAG laser has also been shown to create a potential reflux barrier in dogs. McGouran RCM and Galloway JM 1990, Gastrointest Endosc 36:531-2. Recently, Harrison et al described a method of forming a flap valve at the GEJ by creating an intussusception of esophagus into stomach. U.S. Patent 5,403,326. LoCicero disclosed an endoscopic method for reducing gastroesophageal reflux in U.S. Patent 5,887,594.

US-A-5,897,562 discloses instrumentation for transoral treatment of gastroesophageal reflux disease.

WO-A-99/22649 discloses an endoscopic surgical instrument. US-A-4 880 015 discloses an apparatus according to the preamble of claim 1.

### SUMMARY

According to the invention, an apparatus includes an elongated member configured for transoral placement into the stomach, and a distal end effector including first and second members configured to engage stomach tissue, e.g., stomach tissue beyond the gastroesophageal junction. The first and second members are movable relatively toward one another generally in a first plane, and characterised by the distal end effector being movable relative to the elongated member in a second plane generally transverse to the first plane, such that the distal end effector is bent out of alignment with the elongated member.

Embodiments of the invention may include one or more of the following features.

The distal end effector includes a third member configured to engage stomach tissue. The third member is movable in a distal direction relative to the first and second members. The third member includes a tissue engaging portion, e.g., a coil having a tissue penetrating tip.

The apparatus includes a tissue securement member for coupling to at least one of the first and second members for securing together tissue engaged thereby. The tissue securement member includes a first part for coupling to the first member for engagement with a first tissue section, a second part for coupling to the second member for engagement with a second tissue section to be secured to the first tissue section, a suture attached to the first part, and a securing element attached to the suture and configured for engagement with the second part when the first and second members are moved relatively toward one another to engage the first and second tissue sections, thereby to secure the second part to the first part. The securing element is configured for deployment from the first member, and the first member includes a deploying element for deploying the securing element from the first member. The first member includes tissue piercing elements defining a channel for receiving securing elements.

The second plane is generally perpendicular to the first plane. The distal end effector is configured for movement between a first position generally aligned with the elongated member and a second position in which the distal end effector has moved in the second plane out of alignment with the elongated member. A cable actuatable from a proximal end of the apparatus and coupled to the distal end effector moves the distal end effector in the second plane. A cable actuatable from the proximal end of the apparatus and coupled to the distal end effector moves the first and second members generally in the first plane.

The elongated member defines a channel for receiving an endoscope. A method of using the invention includes advancing an apparatus including an elongated member transorally into the stomach. The apparatus includes a distal end effector having first and second members configured to engage stomach tissue. The first and second members are movable relatively toward one another generally in a first plane. The method includes then moving the distal end effector relative to the elongated member in a second plane generally perpendicular to the first plane to position the first and second members for engagement with the tissue.

Embodiments of this method may include one or more of the following features.

The first and second members are moved relatively toward one another in the first plane to engage tissue, e.g., stomach tissue beyond the esophageal junction. Moving the first and second members engages a first tissue section with a first securing part and a second tissue section with a second securing part. The first securing part includes a suture attached thereto and a securing element attached to the suture. The method includes moving the securing element into engagement with the second securing part to secure the second securing part to the first securing part. Moving the first and second members causes tissue piercing elements of the first member to pierce tissue. Securing elements are deployed through the tissue piercing elements.

The method further includes piercing the tissue with a third member of the distal end effector prior to engaging the tissue with the first and second members.

According to another method of treatment, this includes engaging a plurality of regions of stomach tissue, e.g., stomach tissue beyond the gastroesophageal junction, with a plurality of members from within the stomach, and moving the plurality of members relatively toward one another to pinch the plurality of regions of stomach tissue together thereby reconfiguring tissue in a vicinity of a gastroesophageal junction.

Embodiments of this method may include one more of the following features. The moving includes forming a bulge in such tissue. The moving includes wrapping stomach tissue around the esophagus or around the gastroesophageal junction. The method includes securing the engaged plurality of regions of stomach tissue together, and the securing includes deploying two securing elements connected by suture through the engaged plurality of regions of stomach tissue. Tissue located between the plurality of regions of stomach tissue is pulled prior to engaging the plurality of regions of stomach tissue.

In an illustrated embodiment, the plurality of members includes first and second members and the moving of the first and second members engages a first tissue section with a first securing part and a second tissue section with a second securing part. The method includes moving a securing element of the first securing part into engagement with the second securing part to secure the second securing part to the first securing part. The moving of the first and second members causes a tissue piercing element of the first member to pierce tissue. The method includes deploying the securing element through the tissue piercing element. The moving of the first and second members causes first and second tissue piercing elements of the first member to pierce tissue, and the method includes deploying first and second elements of the securing element each through one of the first and second tissue piercing elements.

The method includes measuring tissue pressure and yield pressure. The method is effective to treat GERD.

According to another method of treatment, this includes engaging a plurality of regions of tissue with a plurality of members from within the stomach, and moving the plurality of members relatively toward one another to wrap tissue in a vicinity of a gastroesophageal junction around the gastroesophageal junction.

According to another method of treatment, this includes engaging a plurality of regions of tissue with a plurality of members from within the stomach, and moving the plurality of members relatively toward one another to pinch the plurality of regions of tissue together in a non- intussuscepting manner thereby reconfiguring tissue in a vicinity of a gastroesophageal junction.

According to another method of treatment this includes engaging a plurality of regions of tissue with a plurality of members from within the stomach, and moving the plurality of members relatively toward one another circumferentially relative to a circumference of a gastroesophageal junction to pinch the plurality of regions of tissue together thereby reconfiguring tissue in a vicinity of the gastroesophageal junction.

According to another method of reconfiguring tissue in the vicinity of a junction between first and second hollow organs, this includes engaging a plurality of regions of tissue of the first organ with a plurality of members from within the first organ, and moving the plurality of members relatively toward one another to pinch the plurality of regions of tissue of the first organ together thereby reconfiguring tissue in a vicinity of the junction.

Embodiments of this method may include the junction being a region of transition between the organs and includes tissue of both organs. The engaging tissue includes engaging tissue of the first organ beyond the junction.

According to another method of attaching at least a portion of a stomach of a subject to a body structure extrinsic to the stomach, this includes engaging a portion of at least the inner surface of the stomach, manipulating the engaged portion of the stomach to bring the outer surface of the stomach into apposition with at least a portion of a body structure extrinsic to the stomach, and securing the stomach to the structure extrinsic to the stomach.

Embodiments of this method may include one or more of the following features.

The body structure extrinsic to the stomach is selected from the group consisting of a muscle, a ligament, a tendon, fascia, and a bone. The body structure extrinsic to the stomach is an aspect of the diaphragm of the subject. The aspect of the diaphragm is selected from the group consisting of median arcuate ligament, right crus, and left crus. The aspect of the diaphragm is the median arcuate ligament.

Tissue pressure is measured at the GEJ in association with at least one of the steps of engaging, manipulating, and securing. Yield pressure is measured in association with at least one of the steps of engaging, manipulating, and securing. The securing is effective to treat, e.g., a hiatus hernia and GERD. The method includes endoscopic visualization of at least a portion of at least one of the steps of engaging, reconfiguring, and securing.

The instrument of the invention advantageously provides an endoscopic approach to treating GERD that does not require the surgical formation of portals to access the GEJ. The procedure can be performed as an outpatient procedure done under sedation, without general anesthesia being required. The procedure can be performed by gastroenterologists rather than a surgeon, and takes less time, has fewer complications and side-effects and has lower overall procedure costs than surgical methods. The procedure recreates or augments the natural anatomy, and is easily reversible.

Major advantages of the invention as it relates to the treatment of GERD include recreation of normal anatomy, reduced morbidity, increased efficacy, and technical ease in clinical practice. In particular, the method of using the invention reestablishes normal gastroesophageal flap valve anatomy, avoids safety concerns related to methods which involve stapling through the esophagus, avoids possible functional compromise associated with placement of tissue fixation devices directly in sealing surfaces, and can be performed by an endoscopist with the subj ect sedated but not under general anesthesia.

Other features, objects, and advantages of the invention will be apparent from the following detailed description, and from the claims.

### DESCRIPTION OF DRAWINGS

FIG. 1 is a pictorial representation of a stomach in anteroposterior cutaway view in which a gastroscope has been advanced through the lumen of the esophagus into the lumen of the stomach and retroflexed to visualize the GEJ;
FIG. 2 is a pictorial representation of a stomach as in FIG. 1 with a tissue engaging device within the lumen of the stomach;
FIG. 3 is a pictorial representation of a stomach as in FIG. 2, where the tissue engaging device has engaged and invaginated a portion of the stomach wall;
FIG. 4 is a pictorial representation of a sectional view of a stomach looking toward the opening of the esophagus into the stomach, showing two points of tissue engagement and arrows indicating the direction of force to be applied to engaged tissue to create a tissue fold covering the GEJ;
FIG. 5 is a pictorial representation of a sectional view of a stomach looking toward the opening of the esophagus into the stomach, showing the invaginated rectangular tissue fold covering the GEJ;
FIG. 6 is a cross-sectional view of a stomach looking toward the opening of the esophagus into the stomach, showing an invaginated triangular tissue fold covering the GEJ;
FIG. 7 is a pictorial representation of a stomach as in FIG. 3, with an endoscopic tissue securing device also introduced into the lumen of the stomach via the esophagus;
FIG. 8 is a pictorial representation of a stomach in anteroposterior cutaway view following deployment of one tissue fixation device to maintain the invaginated portion of stomach wall;
FIG. 9 is a pictorial representation of a stomach depicting a row of tissue fixation devices;
FIG. 10 is a pictorial representation of a stomach depicting three parallel rows of tissue fixation devices;
FIG. 11 is a pictorial representation of a stomach depicting nonparallel rows of tissue fixation devices;
FIG. 12 is a pictorial representation of a stomach depicting a triangular array of tissue fixation devices;
FIG. 13 is a pictorial representation of a stomach depicting multiple tissue fixation devices in curved rows;
FIG. 14 is a cross-sectional view of a stomach looking toward the opening of the esophagus into the stomach, showing a normal gastroesophageal flap valve;
FIG. 15 is a cross-sectional view of a stomach looking toward the opening of the esophagus into the stomach, showing an invaginated tissue bulge opposite the flap of the gastroesophageal flap valve;
FIG. 16 is a cross-section view of a stomach looking toward the opening of the esophagus into the stomach, illustrating one method of bringing together sealing surfaces and the existing gastroesophageal flap valve;
FIG. 17 is a cross-sectional view of a stomach looking toward the opening of the esophagus into the stomach, showing a method of augmenting an existing gastroesophageal flap valve;
FIG. 18 is a cross-sectional view of a stomach looking toward the opening of the esophagus into the stomach, showing fixation of an invaginated fold of tissue at its base with three tissue fixing devices;
FIG. 19 is a cross-sectional view of a stomach looking toward the opening of the esophagus into the stomach, showing a rectangular invaginated fold of tissue fixed at its base covering the GEJ;
FIG. 20 is a pictorial representation of the stomach of FIG. 1 in which a combination tissue engaging device/tissue securing device has been advanced into the lumen of the stomach via the lumen of the esophagus;
FIG. 21 is a pictorial representation of the stomach of FIG. 20 following creation and apposition of two evaginations of stomach wall;
FIG. 22 is a pictorial representation of the stomach of FIG. 20 in exterior view showing the direction of manipulation of stomach tissue from within;
FIG. 23 is a pictorial representation of the stomach of FIG. 22 showing fixation between evaginations of stomach wall;
FIG. 24 is a pictorial representation of the stomach of FIG. 23 showing fixation of an aspect of the diaphragm between evaginations of stomach wall;
FIG. 25 is a side-elevation view of a preferred embodiment of a tissue engaging, manipulating and fixing device of this invention;
FIG. 26 is a side-elevation, schematic view of a portion of the device of FIG. 25;
FIG. 27 is a pictorial representation of a corkscrew-like tissue engagement device;
FIG. 28 is a pictorial representation of (top) an open-ended tube suction tissue engagement device, (middle) a blind-ended tube suction tissue engagement device, and (bottom) an open-ended and flanged tube suction tissue engagement device;
FIG. 29 is a pictorial representation of a two-part tissue fixation device;
FIG. 30 is a partial side, elevation, schematic view of the device of FIG. 25;
FIG. 31 is a side-elevation view of a preferred embodiment of a tissue engaging, manipulating and fixing device of this invention as in FIG. 25, further including pressure monitoring tubes; and
FIG. 32 is a side, elevation view of the device of FIG. 25 illustrating one position of the arms thereof;
FIG. 33 is a side, elevation view of the device of FIG. 25 showing yet another position of the arms thereof;
FIG. 34 is a side, elevation view of the device of FIG. 25 showing yet another position of the arms thereof;
FIG. 35 is a side, elevation view of the device of FIG. 25 showing another position of the arms thereof;
FIG. 36 is a schematic, partially cut-away side view of a stomach illustrating use of the device of FIG. 25 therein; and
FIG. 37 is a perspective view of a tissue engaging device fitted with opposing rollers.
Fig. 38 is a diagrammatic representation of an instrument in use to reconfigure tissue in the vicinity of the gastroesophageal junction of the stomach;
Fig. 39 shows a tissue fixation device deployed by the instrument of Fig. 1 in use to secure a bulge formed in the tissue;
Fig. 40A is an illustration of the instrument of Fig. 1;
Fig. 40B shows a proximal end of the instrument;
Fig. 40C shows the working channels in a shaft of the instrument;
Fig. 40D is an illustration of a coil assembly of the instrument;
Fig. 41A is a top view of a distal end of the instrument, shown with first and second jaw members in an open position;
Fig. 41B shows the distal end of the instrument located off-axis relative to a shaft of the instrument;
Fig. 42 is a side view of the distal end of the instrument, turned 90 degrees relative to Fig. 41 A;
Fig. 43A is an illustration of a first part of the tissue fixation device of Fig. 39;
Fig. 43B is an illustration of the first jaw member with the first part of the tissue fixation device mounted to the jaw member;
Fig. 44 is an illustration of the second jaw member;
Fig. 45 is an illustration of the tissue fixation device of Fig. 39;
Figs. 46A-46F show the instrument of Fig. 38 in use; and
Fig. 47 is an illustration of tissue secured with the tissue fixation device of Fig. 39.

### DETAILED DESCRIPTION

Various aspects of the invention will now be described with reference to the figures. While this invention has particular application to reducing gastroesophageal reflux, the devices of the invention are not limited to this particular application alone, however, for they can be applied to a stomach as well as to other hollow body organs as discussed below.

### A. Endoscopic Methods for Reconfiguring Tissue Within a Hollow Body Organ.

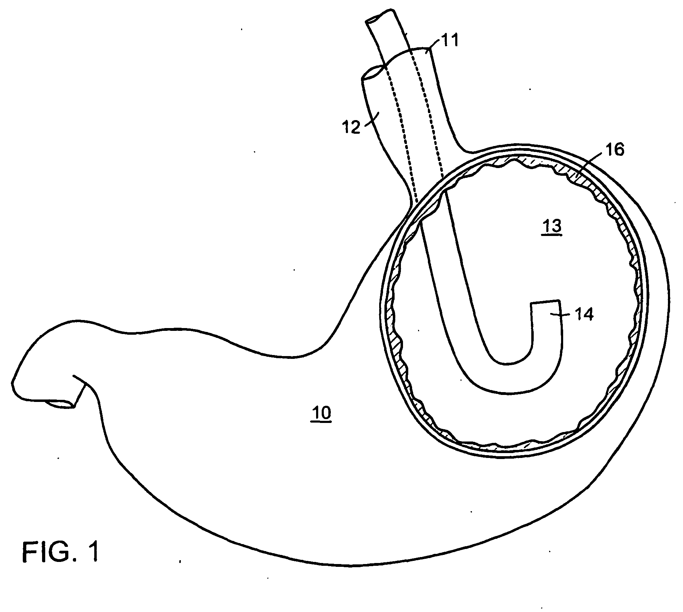

In one aspect, the present invention can be used in endoscopic methods for reconfiguring tissue within a hollow body organ. This method will now be described with particular reference to FIGS. 1-13 which disclose the steps of the method as applied to the stomach for purposes of illustration only. The method may be applied to any hollow organ, as defined below. In a broad sense, the methods include at least the steps of engaging a portion of the inner surface of the hollow organ to be reconfigured, manipulating the engaged portion of tissue to create the reconfiguration, and fixing the manipulated tissue to retain the reconfiguration achieved in the manipulation. The methods may also include the step of endoscopic visualization during all or part of the procedure. Details of the shapes that can be assumed by the reconfigured tissue are discussed below.

As used herein, "endoscopic method" refers to a technique for performing a medical procedure on a subject in which access to the tissue to be treated is gained via an endoluminal approach. In a preferred embodiment an endoscopic method is performed without a contemporaneous invasive approach involving a surgical incision to gain access to the area of treatment. This preferred embodiment embraces use of at least one intravenous catheter for the administration of crystalloid or colloid fluids or medication to the subject, but does not require placement through the abdominal wall of an intraabdominal set of trocars, laparoscope, or the like.

The methods also contemplate gaining access to the interior of a subject's stomach via a gastrotomy or gastrostomy. Such methods, which retain the feature of involving minimally invasive access to tissue to be reconfigured, may be of particular value in situations where access via the esophagus is not possible due to distortion or disruption of normal oropharyngeal or proximal esophageal anatomy. Such methods may also be of particular value when a gastrotomy or gastrostomy is present for other medical reasons, such as for enteral feeding, for example.

As used herein, "hollow organ" refers to an organ of a subject's body which depends for its principal function upon its ability to receive and/or act as a conduit for liquid contents. A hollow organ typically is in fluid communication with another hollow organ and/or with the outside of the body. Many organs of the gastrointestinal and genitourinary tracts are classified as hollow viscus organs. These include stomach, gall bladder, uterus, and bladder. Other hollow organs which act more as fluid passageways include esophagus, small and large intestines, hepatic ducts, cystic duct, common bile duct, pancreatic duct, heart, veins, arteries, vagina, uterine (i.e., Fallopian) tubes, ureters, and urethra.

In the case of a stomach being the hollow organ, "liquid contents" includes any of the following: masticated food, imbibed liquid, chyme, gastric mucus, gastric acid, and other gastric secretions. In other contexts "liquid contents" can also include other body fluids such as intestinal contents, bile, exocrine pancreatic secretions, blood, and urine.

*Endoscopic visualization.* Endoscopic visualization can be used for all at least a part, or none of the procedure. In certain preferred embodiments of the method, the method is performed in conjunction with endoscopic visualization of at least the engaged portion of tissue. Typically, as shown in FIG. 1, a first step in the method includes advancing an endoscope 14 into the interior or lumen 13 of a first hollow organ 10. Preferably, but not necessarily, endoscope 14 is advanced into the interior of first hollow organ 10 by way of a lumen 11 of a second hollow organ 12 in fluid communication with the first hollow organ 10.

Endoscopes are well known in the art. Viewing endoscopic instruments typically are equipped with a lighting element and a viewing element enabling an operator to view the interior of the accessed body cavity. Viewing endoscopic instruments often also include at least one fluid channel suitable for introducing and/or withdrawing a fluid, gas, or medicament, and a working channel suitable to accommodate a remotely operated surgical tool such as a needle, a grasper, a biopsy device, a brush, an electrocautery electrode, and the like. Acceptable viewing elements include fiberoptic-assisted direct visualization, television array, and video array endoscopes. Endoscope 14 can be introduced into lumen 13 of the first hollow organ for at least one aspect of the procedure and removed for at least one other aspect of the procedure. Accordingly, a viewing endoscope 14 can be introduced, removed, and reintroduced for any one or for any combination of steps of the method of the invention.

For the purposes of the invention, a viewing endoscope can be an instrument separate from any other instrument employed in a method using the invention. Alternatively, a viewing endoscope can work cooperatively with at least one other instrument of the invention by, for example, the at least one other instrument's cooperatively positioning the endoscope. In other embodiments, a viewing endoscope can be incorporated into a tissue engaging device, at least a portion of a tissue fixing device, or part of a combined tissue engaging and tissue securing device.

It should be noted that existing flexible endoscopes may not be sufficiently rigid when flexed to serve as the working platform for performing the types of stomach tissue manipulation described below. The types of pushing, pulling, and side-to-side manipulations to be performed in the vicinity of the opening of the esophagus into the stomach require a degree of mechanical leverage for which a retroflexed gastroscope normally cannot serve as an adequate fulcrum. To perform these manipulations in a stomach, an endoscope may be used, for viewing purposes, in conjunction with a specially structured instrument such as described below. In applications where rigid endoscopes may be used, the methods may be practiced without such specially structured instruments.

The method may be performed at least in part with non-endoscopic visualization of the tissue engaged. Non-endoscopic methods of visualization include techniques well known in the art, such as, without limitation, fluoroscopy, either with or without the use of a suitable radiographic contrast agent, and ultrasound. In some embodiments the procedure can be performed without any endoscopic visualization.

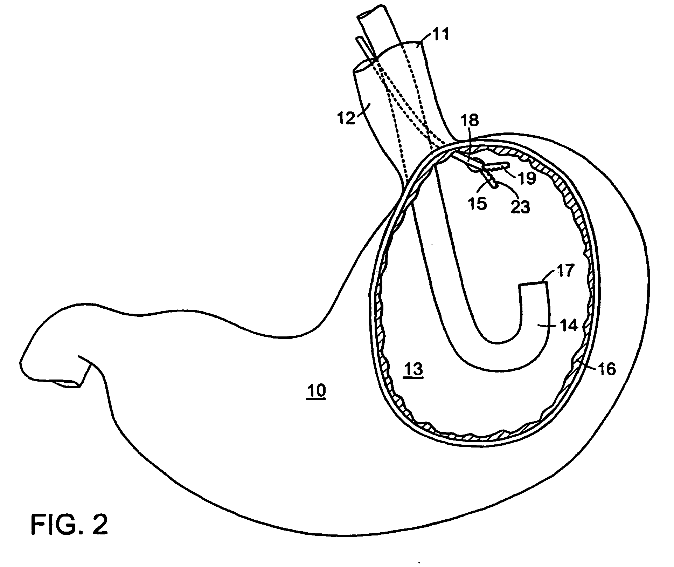

*Engaging.* An early step of this aspect of the invention is engaging the selected portion of an inner surface 16 of first hollow organ 10, as shown in FIG. 2.

As used herein, the term "engaging" refers to an act of reversibly penetrating, gripping, tweezing, holding, pressing, clamping, sucking, or otherwise contacting a tissue in a mechanical fashion so as to establish a physical connection with the tissue. In certain preferred embodiments of the invention the engagement of tissue occurs reversibly and essentially atraumatically.

For purposes of this invention, an endoscopic tissue engaging device is understood to have a proximal end and a distal end interconnected by an elongate portion of suitable length and rigidity to permit an operator, in contact with and control of the proximal end, to gain remote access to the interior of a body cavity with the distal end of the endoscopic tissue engaging device. Furthermore, the operator of an endoscopic tissue engaging device is understood to be able to actuate a tissue engaging element disposed at the distal end by manipulation of at least one aspect of a controlling mechanism disposed at the proximal end and operatively connected to the tissue engaging element disposed at the distal end.

The tissue engaging device in some aspects can be a separate instrument unto itself. In other embodiments the tissue engaging device can be used in combination with another endoscopic instrument. In yet other embodiments the tissue engaging device can be an element of a combination endoscopic instrument. In a preferred embodiment the tissue engaging device is an element of an endoscopic instrument which also incorporates a tissue securing device (see below).

As used herein, an "engaged portion" shall refer to a segment of tissue actually engaged by a device used to engage the tissue.

In certain preferred aspect the engaged portion involves just the inner lining of the first hollow organ 10. For example the engaged portion can involve only the mucosa in a stomach. In other embodiments the engaged portion can involve the inner lining and at least one additional tissue layer of first hollow organ 10. Again with reference to the stomach, the reconfigured portion can involve the mucosa and at least one layer of muscular wall, up to and including the full thickness of the stomach wall.

In certain preferred aspects the tissue engaging device can engage tissue in a reversible and essentially atraumatic manner. Engagement of tissue in such embodiments is effective for performing subsequent steps of the method but also allows release of engaged tissue in a manner which causes little or no disruption of tissue integrity.

For example, in a most preferred aspect the tissue engagement device includes a novel corkscrew-type element, described below. Even though the sharpened end of the spiral corkscrew-type element pierces in order to engage tissue, when the spiral is removed by unscrewing it from tissue, it leaves a single discrete point of penetration which self-seals in the extremely pliable tissue lining of the stomach, much as does a hole made in the same tissue with a hypodermic needle.

In yet other aspects the tissue engaging device can be a known clamping device. Examples of suitable endoscopic clamping devices are well known in the art, including, without limitation, endoscopic alligator grasping forceps (see FIG. 2), forked jaw grasping forceps, rat tooth grasping forceps, three-prong grasping forceps, tripod grasping forceps, fenestrated cup forceps, and ellipsoid fenestrated forceps. As used herein, each such endoscopic clamping device is considered to engage a single portion of tissue, i.e., all tissue contacted by the various jaws of a single clamping device is considered as a single point of tissue engagement.

In other preferred aspects the tissue engaging device can be a novel suction device, as described below. Tissue is engaged when contacted with suction and released atraumatically when the suction is broken at a point other than the point of tissue engagement.

According to the above method, the tissue engaging device can engage tissue for the purposes of side-to-side manipulation, twisting, pushing, or retracting tissue. In yet another method a tissue engaging device may be the sharpened end of, for example, at least one leg of a surgical staple. According to this method, the tissue engaging device can engage tissue for the purposes of side-to-side manipulation, twisting, or pushing, but not for retracting tissue.

In a preferred method a tissue engaging device may be incorporated into a tissue manipulating device. In this embodiment, the elongate portion of the tissue engaging device is further structured to permit manipulation of tissue engaged by atraumatic grasping, suction, or piercing as above. In a most preferred method, as discussed below, a novel single instrument incorporating both a tissue engaging and tissue manipulation device is structured to permit independent engagement of tissue at two or more points, and to permit manipulation of at least two points of tissue with respect to each other in any direction in three-dimensional space. The two or more independent points of tissue engagement typically are separated by at least 1 cm prior to tissue engagement.

In a preferred aspect, an endoscopic tissue engaging device 18 is advanced into lumen 13 of first hollow organ 10, preferably via a lumen 11 of second hollow organ 12. In FIG. 2 first hollow organ 10 is shown as a stomach and second hollow organ 12 is shown as an esophagus in fluid communication with organ 10. Distal end 17 of endoscope 14 and a distal end of tissue engaging device 18 are shown in position in FIG. 2 after they have been advanced into lumen 13 of organ 10 via the lumen 11 of organ 12. The related portion of inner surface 16 of first hollow organ 10 is engaged with endoscopic tissue engaging device 18, which is described below.

In one preferred method engaging is accomplished by gripping tissue with a known jawed forceps device 18 as illustrated schematically in FIG. 2. Device 18 includes opposed jaws 15 and 19 having teeth 23 or the like. The engaging force must be sufficient to maintain physical connection with the engaged portion of tissue when a tissue-deforming torque, push, or retraction is applied to the engaged tissue via the tissue engaging device, while at the same time the force distribution is sufficient to avoid piercing, tearing, or cutting the surface of the engaged portion.

In certain preferred methods the engagement involves simultaneous engagement of at least two distinct sites. This effect can be achieved by simultaneously applying at least two tissue engagement devices, e.g., two separate endoscopic forceps clamps, or applying a single tissue engagement device designed to engage tissue simultaneously at distinct sites. A device of the latter type is described below.

Release of the engaged portion is necessary in order to remove the engaging device from the hollow organ of the subject after having engaged the tissue. The engaged portion typically will participate in the reconfigured portion; i.e., the reconfigured tissue will typically comprise in some aspect, be it at a basal, apical, or intermediate position relative to the reconfigured portion as a whole, the tissue actually engaged by the engaging device in the course of reconfiguring.

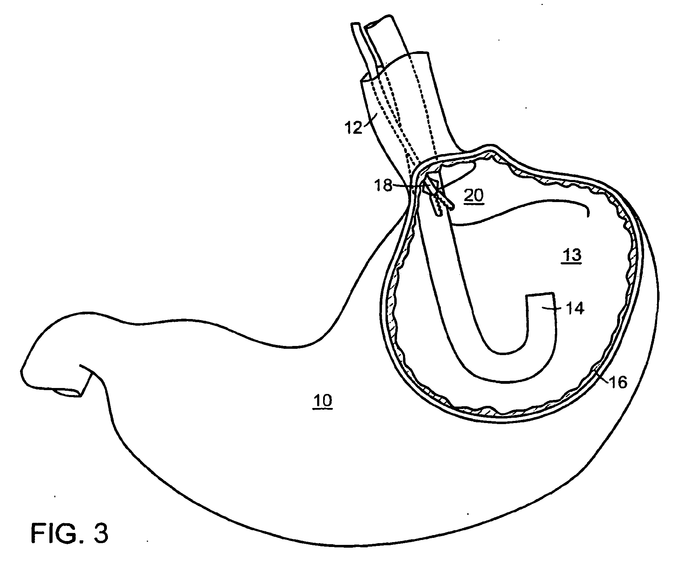

*Manipulating.* In a subsequent step, the engaged portion of inner surface 16 of first hollow organ 10 is manipulated to reconfigure at least a portion of first hollow organ 10, as shown in FIG. 3. Inner surface 16 of first organ 10 is manipulated by device 18 to create a reconfigured portion 20. In the manipulating step a physical force is applied by device 18 to the engaged portion of tissue of inner surface 16 effective for pushing, pulling, twisting, rolling, folding, gathering, or otherwise displacing tissue from its original position and/or configuration prior to application of such force. In preferred embodiments, tissue in adjacent continuity with the portion actually engaged will undergo at least some degree of physical deformation from its original conformation in proportion to the magnitude and direction of force applied to the engaged portion. Manipulation of engaged tissue may be used to create an invagination, an evagination, or a combination of invagination and evagination of at least inner layer 16 of first hollow organ 10.

In one embodiment, manipulation of the engaged portion of inner surface 16 of first hollow organ 10 is achieved by applying traction force or torquing force to create reconfigured portion 20, which is an invagination. Traction force can be linear, such as achieved by pulling. Alternatively, traction force can be nonlinear, such as can be achieved by winding engaging tissue onto a spool.

As used herein, "invaginated portion" or "invagination" refers to a region of tissue displaced toward the interior cavity of the hollow organ as a combined result of engaging and manipulating. The particular shape assumed by the invaginated portion will depend on factors including the geometry of the engaged portion, the anatomy of the engaged organ, the plasticity of the segment of the organ engaged, and the direction and magnitude of the force applied.

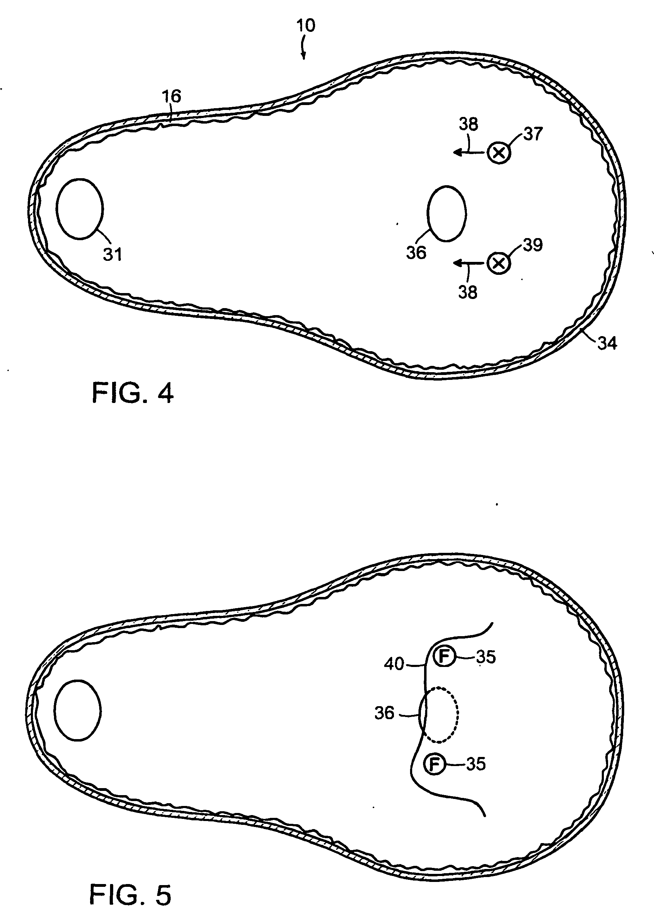

Examples of forming invaginations that assume the shape of a flap or fold are shown in FIGS. 4-6. FIG. 4 depicts a cross-sectional view of a stomach looking toward the opening of the esophagus into the stomach 36. Also shown in FIG. 4 is the opening of the duodenum into the stomach 31. In FIG. 4, inner surface 16 of stomach 10 is engaged at two points 37 and 39 on one side of opening of the esophagus into the stomach 36, flanking opening of the esophagus into the stomach 36. The engaged tissue is then manipulated in the direction indicated by the arrows 38, i.e., in a direction generally toward and across the opening of the esophagus into the stomach 36 from points of engagement 37 and 39. FIG. 5 depicts a generally rectangular flap 40 created by the engaging and manipulating steps shown in FIG. 4. The flap 40 is fixed at points 35, which are in the direction of or across the aperture of opening of the esophagus into the stomach 36 relative to points of engagement 37 and 39. The opening of the esophagus into the stomach 36 is at least partially covered by rectangular flap 40. Two tissue fixation devices each pass through at least two layers of stomach wall: the layer or layers forming fold 40 and at least the lining 16 of the stomach wall near the opening of the esophagus into the stomach 36. The size and tightness of the fold depend on the location of points of fixation 35 relative to the points of engagement 37 and 39 and the position of the opening of the esophagus into the stomach 36.

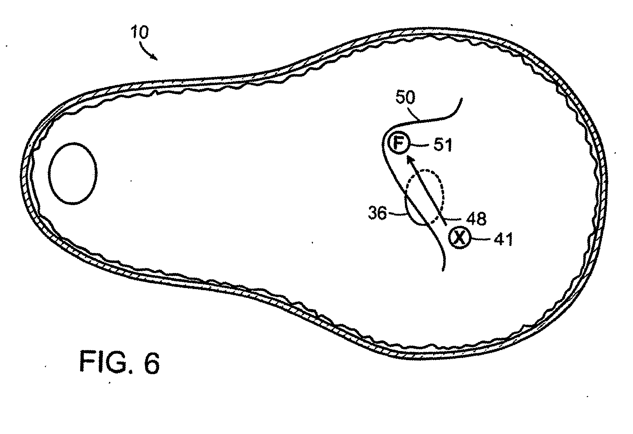

A method of making an alternative flap configuration is depicted in FIG. 6. Inner surface 16 of stomach 10 is engaged at a single point 41 near opening of the esophagus into the stomach 36. The engaged tissue is then manipulated in the direction indicated by the arrow 48, i.e., in a direction generally toward and across the opening of the esophagus into the stomach 36 from point of tissue engagement 41. FIG. 6 depicts a triangular flap 50 created by the engaging and manipulating steps shown in FIG. 6. The opening of the esophagus into the stomach 36 is at least partially covered by flap 50. The fold 50 is fixed at a single point 51 generally across the opening of the esophagus into the stomach 36 from point of tissue engagement 41. A single tissue fixation device passes through at least two layers of stomach wall: the layer or layers forming the fold 50 and the lining 16 of the stomach wall near the opening of the esophagus into the stomach 36. The size and tightness of the fold 50 depend on the location of the point of fixation 51 relative to the point of engagement 41 and the position of the opening of the esophagus into the stomach 36.

It is emphasized that the rectangular and triangular shapes described above are highly schematic. Due to the plasticity of the tissue involved, the actual configuration of tissue achieved by such methods may not appear so definitively rectangular or triangular. Nevertheless, it is useful to think in terms of these shapes or structures for the purposes of conceptualizing the methods used to achieve the desired functional effects, i.e., the inhibition of reflux.

Reconfigured portion 20 can assume any of a range of alternative shapes, including without limitation, a flap, a fold, a bulge, a mound, a ridge, a roll ("jellyroll"), a tube, a papilla, or a cone. The mechanics of mobility of the shaped tissue depend on factors including, for example, the size, shape, thickness, radius, position, and composition of the involved tissue, as well as the shape of the fastener or fasteners, and the placement position of the fastener or fasteners.

In certain methods, the invagination forming reconfigured portion 20 can take the shape of a tissue bulge. As used herein, "tissue bulge" refers to a gathered up or heaped up mound of tissue with a base and an apex relative to the contour of tissue from which it arises. The circumference at its base can be irregular or it can be substantially regular, e.g., substantially elliptical, substantially circular, substantially triangular, or substantially rectangular. A tissue bulge resembles, when viewed from within the hollow organ, a lump or a mass or a papilla. A mound of tissue forming a tissue bulge is to be distinguished from a flap or fold of tissue in that it need not have distinct opposing surfaces or sides. As viewed from within the hollow organ, a tissue bulge can be smooth, dimpled, or furrowed.

According to an embodiment of this method, manipulation can entail bringing into apposition at least two points of tissue which are independently engaged by at least one tissue engaging device.

According to yet another aspect of the method, combinations of tissue invaginations are created. Thus for example a flap and a bulge can be created in combination. Other embodiments include, without limitation, at least two flaps; at least two bulges; at least two rolls; a roll and a bulge; etc. Combinations of tissue invaginations can be created essentially contemporaneously or consecutively.

According to yet another embodiment of this aspect of the method, manipulation of the engaged portion of inner surface 16 of first hollow organ 10 can be achieved by applying a leading or pushing force so as to create, from within, an outward protrusion of the first hollow organ (not shown). According to this method reconfigured portion 20 is an evagination rather than an invagination. An evaginated portion can assume any of a number of shapes as viewed from the exterior of the hollow organ, including, without limitation, a bulge, a lump, a ridge, a flap, a fold, a tube, a horn, and a cone. As also viewed from the exterior of the hollow organ, a tissue evagination can be smooth, dimpled, or furrowed. The circumference at its base can be irregular or it can be substantially regular, e.g., substantially elliptical, substantially circular, substantially triangular, or substantially rectangular. The method also contemplates the formation of a plurality of evaginations, which may be created either simultaneously or sequentially. At least one evagination can be combined with at least one invagination.

In some embodiments reconfigured portion 20 involves just the inner lining of the first hollow organ 10. For example reconfigured portion 20 can involve only the mucosa in a stomach. In other embodiments reconfigured portion 20 can involve the inner lining and at least one additional tissue layer of first hollow organ 10. Again with reference to the stomach, the reconfigured portion can involve the mucosa and at least one layer of muscular wall, up to and including the full thickness of the stomach wall.

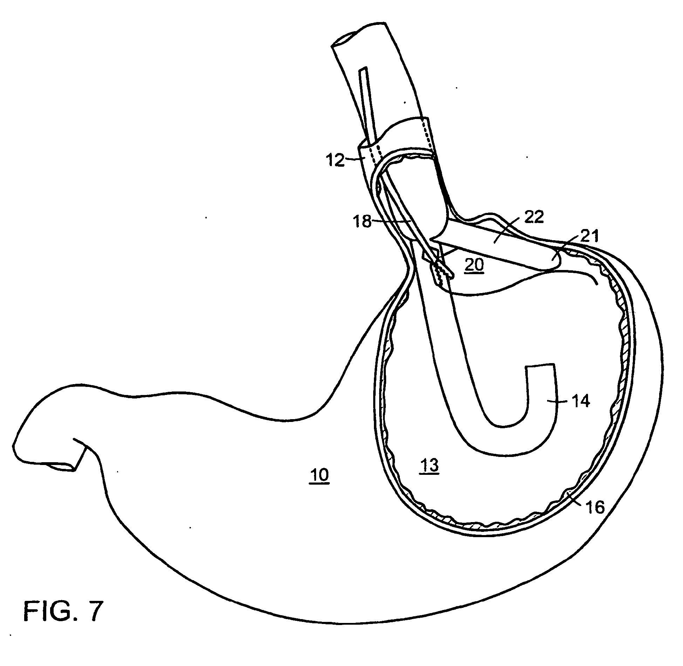

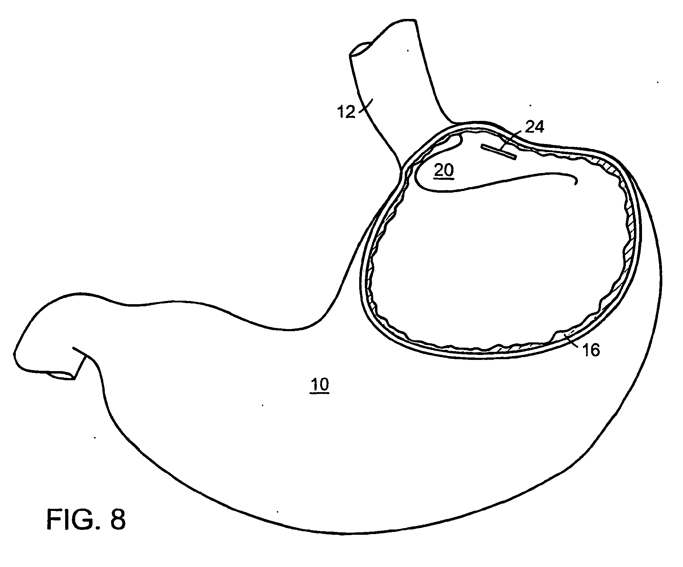

*Securing.* After the manipulating step, a subsequent step involves permanently securing reconfigured portion 20 of first hollow organ 10 to effect a substantially permanent retention of the shape of reconfigured portion 20, as shown in FIGS. 7 and 8. While reconfigured portion 20 of organ 10 is maintained under control of the operator through the manipulating force applied to the engaged portion of tissue via the tissue engaging device 18, the operator causes a distal effector end 21 of a tissue securing device 22 (described below) to come into contact with reconfigured portion 20. Distal effector end 21 of tissue securing device 22 includes at least one biocompatible tissue fixation device 24 (described below) and is structured for application of at least one biocompatible tissue fixation device 24 into reconfigured portion 20. Tissue securing device 22 is advanced into lumen 13 of first hollow organ 10 before, along with, or after the tissue engaging device 18; thereafter, tissue securing device 22 is actuated to apply at least one biocompatible tissue fixation device 24 to permanently secure or fix the shape of reconfigured portion 20.

As used herein, "permanently secure" refers to directed placement of at least one biocompatible tissue fixation device effective for stabilizing tissue in a desired position. Permanently securing is preferably accomplished from within lumen 13 of first hollow organ 10. "Permanently" means for as long as there is clinical utility. This definition contemplates the intentional, active removal of a tissue fixation device by a practitioner based on his professional judgment. When permanently securing is accomplished by applying at least one resorbable tissue fixation device, the invention contemplates the formation of tissue adhesion arising at the site of or as a result of the presence of the applied resorbable tissue fixation device during the time such device remains intact. "Permanently securing" contemplates that such tissue adhesion is effective to maintain the configuration of the reconfigured tissue after resorption of the resorbable tissue fixation device.

According to a preferred embodiment of this method, securing an invaginated portion of a hollow body organ in a new position preferably does not involve tissue extrinsic to the first hollow body organ. Thus the method a preferably does not involve securing tissue of the first hollow body organ 10 to tissue of a second hollow body organ 12 in fluid communication with the first. In the particular instance where the first hollow body organ 10 is a stomach and the second hollow organ 12 is an esophagus, according to this method, stomach tissue is secured only to stomach tissue, and not to tissue of the esophagus.

The tissue fixation device 24 used in this method is a mechanical entity useful for stabilizing a particular configuration of at least one tissue. A tissue fixation device 24 is deployed or applied to a tissue by a tissue securing means 22 structured to deliver the tissue fixation device 24.

The tissue securing device 22 is understood to have a proximal end and a distal end 21 interconnected by an elongate portion of suitable length to permit an operator, in contact with and control of the proximal end, to gain remote access to the interior of a body cavity with the distal end 21 of the endoscopic tissue engaging device 22. Furthermore, the operator of an endoscopic tissue engaging device 22 is understood to be able to actuate an effector element disposed at the distal end 21 by manipulation of at least one aspect of a controlling mechanism disposed at the proximal end and operatively connected to the effector element disposed at the distal end 21. The effector element can be structured to deliver at least one tissue fixation device 24, tissue adhesive, or radio frequency (RF) energy into tissue contacted with the effector element.

The tissue securing device can be a separate instrument unto itself. In other embodiments the tissue securing device can be used in combination with another endoscopic instrument. Moreover the tissue securing device can be an element of a combination endoscopic instrument. In a preferred aspect, the tissue securing device is an element of an endoscopic tissue shaping instrument which also incorporates a tissue engaging device.

In a preferred aspect tissue fixation device 24 is a biocompatible staple and tissue securing device 22 is an endoscopic surgical stapler. Examples of surgical staplers are well known in the art, including those disclosed in U.S. Patent Nos. 5,040,715 and 5,376,095. Stapling devices can be anviled or one-sided. A biocompatible staple is commonly made of non-resorbable material such as titanium or stainless steel, but other materials, including resorbable materials, are embraced by the invention. Other tissue fixation devices 24 can be a biocompatible clip, tack, rivet, two-part fastener, helical fastener, T-bar suture, suture, or the like, examples of which are well known in the art. Preferably, tissue fixation device 24 is non-resorbable.

In certain methods, tissue fixation device 24 penetrates only an internal layer of first hollow organ 10. The internal layer can be, for example, the mucosa lining the interior of the stomach. Alternatively, tissue fixation device 24 penetrates both internal and at least one additional layer of first hollow organ 10. The at least one additional layer can be, for example, a muscle layer of the stomach wall. The combined inner layer and at least one additional layer constitute either a partial-thickness layer or a full-thickness layer. The securing step of this method includes, for example, fixation of an inner layer to a partial-thickness layer. This step also includes, for example, fixation of an inner layer to a full-thickness layer. In certain other methods, in the securing step, the tissue fixation device penetrates (1) a partial-thickness layer and (2) a full-thickness layer, or two full-thickness layers of the first hollow organ 10. This latter securing step fixes, for example, a full-thickness invagination in which two or more distinct regions of the exterior surface of the first hollow organ are brought into apposition (not shown).

In yet other securing steps where more than one tissue fixation device 24 is used, there can be any combination of tissue fixation devices 24 penetrating any combination of layers. For example a first tissue fixation device 24 penetrating a partial-thickness layer can be used in combination with a second tissue fixation device penetrating an inner layer alone. As another example, a first tissue fixation device 24 penetrating both an inner layer and a partial-thickness layer can be used in combination with a second tissue fixation device 24 penetrating an inner layer and a full-thickness layer. These and other possible combinations of tissue fixation devices used to fix combinations of tissue layers are intended to be encompassed by the invention.

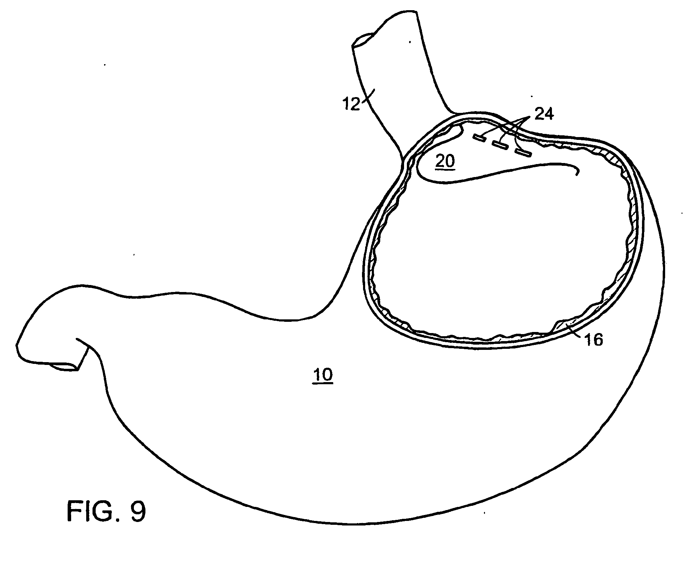

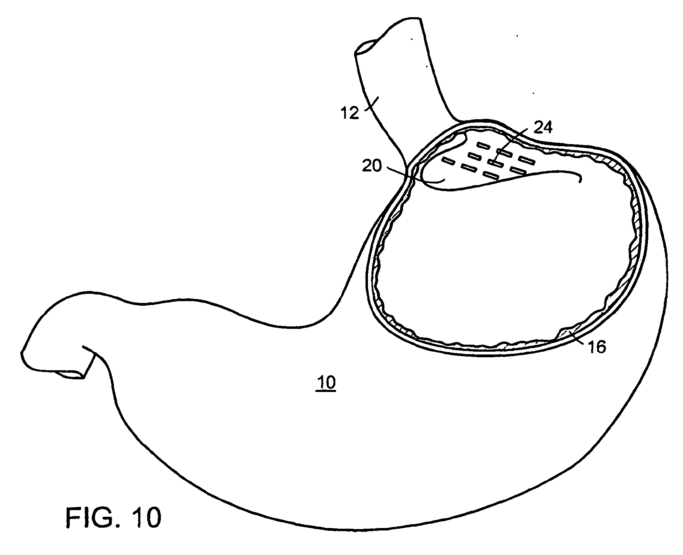

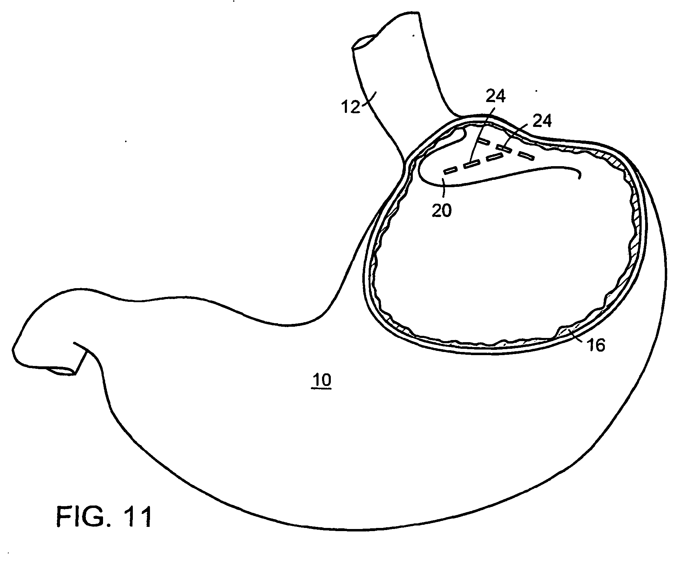

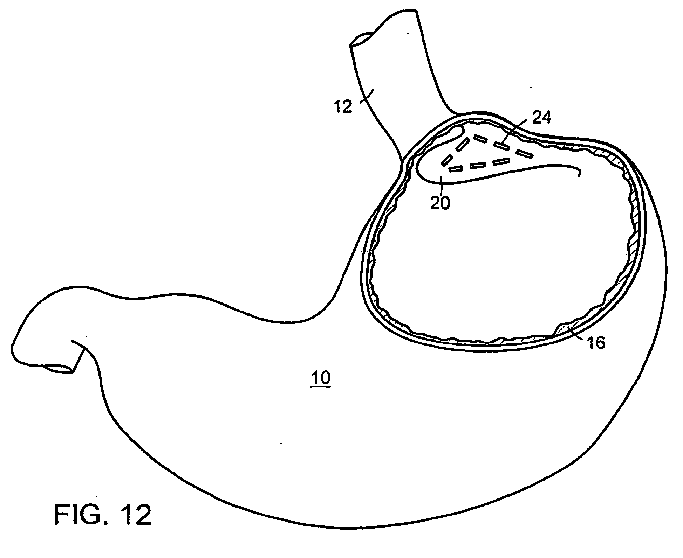

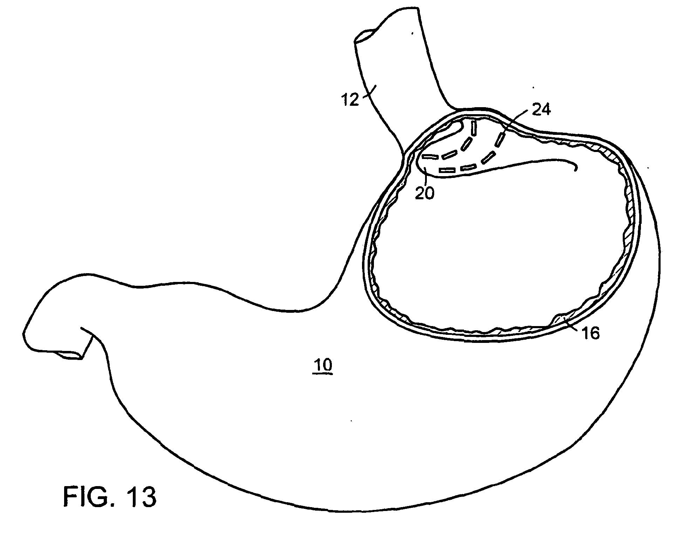

FIGS. 9-13 illustrate various geometric patterns that can be used when the at least one tissue fixation device 24 is a biocompatible staple. When more than one tissue fixation device 24 is deployed, the tissue fixation devices 24 can be delivered sequentially or simultaneously. Examples of geometric patterns include a line (FIG. 9); two or more parallel lines (FIG. 10); two or more nonparallel lines, including a "T" (FIG. 11) and a cross; at least one polygon, including a triangle (FIG. 12); at least one arc, including two or more curves (FIG. 13); at least one circle. The purposes of alternate configurations are to spread the stresses due to fixation over a greater area of tissue; provide a fail-safe situation, i.e., maintain fixation even if one of the tissue fixation devices should fail; create and maintain tissue shape and positioning for optimal clinical effect; and encourage healing, by creating multiple holes in tissue, causing bleeding or fibrocyte migration.

Achievement of the desired reconfiguring of tissue can require two or more cycles of engaging, manipulating, and securing. For example, in a particular instance the desired size or shape to be effected might not be fully achieved in a single cycle of engaging, manipulating, and securing. The discloses method also contemplates releasing the engaged portion of the first hollow organ 10 and optionally re-engaging that portion or engaging another portion and then manipulating and permanently securing the portion thus engaged.

The shape of tissue assumed by the secured, reconfigured portion 20 may be effective to restrict flow of liquid contents of the first hollow organ 10 into the second hollow organ 12, while allowing normal flow antegrade from the second hollow organ 12 into the first hollow organ 10. Examples of undesired flow from one hollow organ into a contiguous second hollow organ include gastroesophageal reflux, reflux of urine from the urinary bladder retrograde into a ureter, regurgitant blood flow from one chamber to another within the heart, and blood flow through an atrial or ventricular septal defect.

The permanently secured reconfigured portion 20 preferably is effective to restrict reflux of contents of the first hollow organ 10 into the second hollow organ 12. Reconfigured portions may be a valve that hinders or restricts passage of contents from organ 10 to organ 12. Preferably the valve operates as a one-way valve. In a preferred embodiment of the invention the valve created to accomplish the objectives is a flap valve. Examples of such valves occurring naturally in humans include aortic, mitral, pulmonary, and tricuspid valves of the heart, the gastroesophageal flap valve, the ileocecal valve, the epiglottis, and valves in veins. Flap valves are also normally found at the junction between the urinary bladder and the ureters. Other types of valves include nipple valves and multi-leafed valves.

In the case of the stomach it is most desirable that any valve interconnecting the stomach and the esophagus function effectively to restrict the flow of gastric contents into the esophagus under normal circumstances. The ideal valve should function in such a manner so as to permit, under appropriate circumstances, release of gas from the stomach into the esophagus, regurgitation of stomach contents into the esophagus, and interventional aspiration of stomach contents. In the normal individual the gastroesophageal flap valve achieves this desired degree of functional discrimination.

The desired effect of the resulting secured, reconfigured portion 20 includes at least one of the following: reduction in the frequency of unwanted backflow; reduction in the volume of unwanted backflow or reflux; reduction of symptoms related to unwanted backflow or reflux; and increasing yield pressure between the first hollow organ 10 and the second hollow organ 12. Any such desired effect is measured relative to reflux under similar circumstances, e.g., in relation to recumbency, inversion, coughing, sneezing, etc., before the combined steps of reconfiguring and securing. Any such effect is achieved by the ability of the secured, reconfigured portion 20 to impede flow of liquid across a junction from the first hollow organ 10 into the second hollow organ 12, such as the GEJ proximal to opening of the esophagus into the stomach 36.

In a preferred method the secured, reconfigured portion 20 reduces the frequency of episodes of unwanted backflow by at least 50 percent. Most preferably the frequency of unwanted backflow episodes is reduced by about 100 percent.

In another preferred method the secured, reconfigured portion 20 reduces the volume of unwanted fluid backflow by at least 20 percent. In a more preferred embodiment the volume of unwanted fluid backflow is reduced by at least 50 percent.

In another preferred method the secured, reconfigured portion 20 is effective for increasing the competence of the GEJ. As used herein, "competence of the GEJ" refers to the ability of the GEJ to limit the flow of contents of the stomach into the esophagus while allowing the normal passage of food from the esophagus to the stomach. A fully competent GEJ would completely limit the flow of contents of the stomach into the esophagus while allowing the normal passage of food from the esophagus to the stomach.

As used herein, the words "symptoms of reflux" refer to subjective experiences of a subject as well as objective clinical signs attributable to backflow of contents of a distal hollow organ into the lumen of a proximal hollow organ in fluid communication with the first. In a preferred method the symptoms of reflux are related to gastroesophageal reflux.

As used herein, "effective to reduce symptoms of reflux" refers to substantially reducing the frequency, number, and/or severity of symptoms arising as a result of episodic or chronic reflux. In a preferred method the frequency of symptoms of reflux is reduced by at least 50 percent. In another preferred method the severity of symptoms of reflux is reduced by at least 50 percent. In yet another method the number of symptoms of reflux is reduced by at least one.

The secured, reconfigured portion 20 also may be effective for increasing the yield pressure of the junction connecting first hollow organ 10 to second hollow organ 12, such as the GEJ proximal to the opening of the esophagus into the stomach 36. As used herein, the term "yield pressure" refers to the intraluminal pressure of first hollow organ 10 which overcomes a pressure gradient otherwise maintained between first hollow organ 10 and second hollow organ 12. In other words, the yield pressure is the change in pressure which is sufficient to cause flow of contents of first hollow organ 10 into the lumen 11 of the second hollow organ 12. As applied to the yield pressure of the GEJ, yield pressure is the maximum pressure reached inside the stomach prior to refluxive flow when it is infused with gas or liquid, minus the pressure at rest in the stomach. Normal yield pressures of the GEJ fall within the range of 7-15 mm Hg in healthy human subjects (McGouran RCM et al.1988, Gut 29:275-8; Ismail T et al. 1995, Br J Surg 82:943-7), ≤5 mm Hg in subjects with GERD (McGouran RCM et al 1988, *supra;* McGouran RCM et al. 1989, Gut 30:1309-12; Ismail T et al. 1995, *supra*), and >14 mm Hg in subjects with GERD following successful reflux surgery (McGouran RCM et al. 1989, *supra;* Ismail T. et al. 1995, *supra*).

As used herein, "effective to increase yield pressure" refers to an objectively measurable increase in the yield pressure over the pretreatment yield pressure. In a preferred method, the yield pressure is increased to at least 75 percent of normal. The method can include but does not require objective measurement of an increase in yield pressure.

Bench testing was conducted using an excised pig stomach and attached esophagus to demonstrate the principle of creating a bulge to prevent gastroesophageal reflux. The duodenum was clamped, an incision was made in the greater curvature of the stomach, and the stomach was inverted and filled with water. Water was observed to flow under the force of gravity from the stomach to the esophagus in a steady stream. A bulge was made in the wall of the stomach within one inch of the opening of the esophagus into the stomach. The bulge was fixed in place with a staple. The stomach was then refilled with water. No water was observed to flow under the force of gravity from the stomach to the esophagus following this procedure. A one-half inch diameter cylinder was passed through the esophagus and opening of the esophagus into the stomach both before and after creation of the bulge, indicating that the bulge did not close the lumen of the opening of the esophagus into the stomach.

To demonstrate the in vivo effect of creating a bulge on yield pressure of a stomach, a pig was placed under general anesthesia and surgical access to the stomach was gained via an abdominal incision. Two small punctures were made in the stomach, through which a tube for saline inflow was placed into one, and a pressure-monitoring catheter was placed into the other. Purse-string sutures were placed around each incision to secure the tubes and seal the stomach tissue to prevent leakage. After clamping the pylorus, saline was infused into the stomach through the inflow tube until the stomach was full. The stomach was then squeezed by hand and the maximum pressure obtained was observed on equipment attached to the pressure monitoring catheter. Average maximum yield pressure obtained was 32 mm Hg.

The stomach was drained and an incision was made in the wall of the stomach to provide access for instrumentation into the stomach. A bulge was created within 25.4 mm (one inch) of the opening of the esophagus into the stomach and fixed in position with a staple. The incision was closed with suture and the stomach refilled with saline. The stomach was then squeezed again by hand and the maximum pressure obtained observed as before. Average maximum yield pressure obtained after creation of the bulge was 57 mm Hg. Yield pressure thus increased nearly 80 percent over baseline.

### B. Endoscopic Methods for Reconfiguring Tissue Within a Stomach to Treat GERD.

The invention can be used in endoscopic methods for reconfiguring tissue within a stomach to treat GERD. The methods are based upon the observation that a flap of stomach tissue covers the aperture of the esophagus as it enters the stomach, forming a flap valve which provides an effective barrier to reflux of liquid contents of the stomach. An effective flap valve functions as a one-way valve allowing free passage of swallowed liquids and solids from the esophagus into the stomach, but not *vice versa,* while permitting appropriate escape of gas from the stomach into the esophagus, e.g., during belching.

As used herein, a "flap valve" has an aperture and at least two sealing surfaces which, when properly apposed, effectively close the aperture. In a preferred method, at least one of the sealing surfaces is provided by a mobile flap or ridge of tissue. In its closed position, the sealing surface of the flap contacts at least one other sealing surface, including either another flap or a valve seat, in such a manner as to form an effective closure or seal around the aperture.

In certain preferred embodiments a competent flap valve can function as a one-way valve, favoring flow past the valve in one direction and limiting flow past the valve in the opposite direction. As applied to a stomach, a competent flap valve should favor free flow of swallowed materials from esophagus 12 into stomach 10 and limit free flow of liquid contents from stomach 10 into esophagus 12. In a normal subject such a flap valve opens to permit a swallowed bolus to pass from esophagus 12 into stomach 10, but the flap valve otherwise normally remains closed, preventing reflux of liquid contents from stomach 10 into esophagus 12.

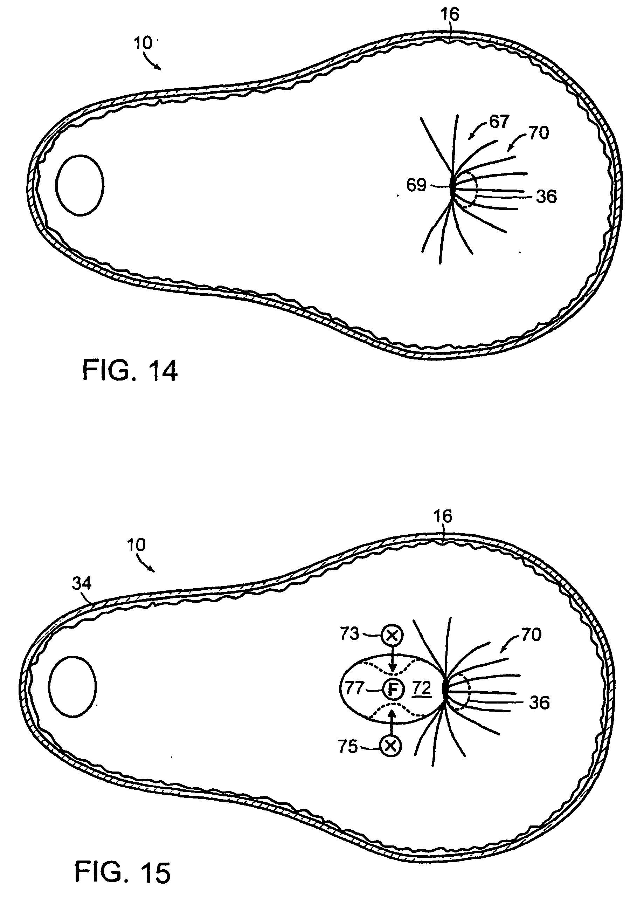

FIG. 14 depicts the configuration of a normal gastroesophageal flap valve 70 in a stomach 10 having an inner surface 16. Here flap portion 67 and valve seat 69 furnish the requisite sealing surfaces. In this view taken from the perspective of the interior of the stomach looking toward the opening of the esophagus into the stomach 36, the flap portion 67 of valve 70 is to the right, covering opening of the esophagus into the stomach 36, and the seat 69 of the valve is to the left of and beneath the covering flap. As used herein in reference to a stomach 10, a flap valve 70 shall be considered to "cover" the opening of the esophagus into the stomach 36 even though flap valve 70, being within the stomach 10, is caudal or inferior to the opening of the esophagus into the stomach 36.

Two factors may make a flap valve 70 incompetent. One factor is the absence of a sufficient flap of tissue 67. The opening of the esophagus into the stomach 36 cannot be effectively closed if a sufficient flap portion 67 is not present to form a seal against at least one other sealing surface 69. The flap 67 can be either be too small or simply absent altogether. The second factor is the effective apposition of sealing surfaces. The opening of the esophagus into the stomach 36 cannot be effectively closed, even if a sufficient flap portion 67 is present, if the sealing surfaces cannot be properly apposed. As used herein, sealing surfaces are properly "apposed" when their mutual contact causes the surfaces to form an effective barrier to reflux.

In clinical application, the existence or appearance of a gastroesophageal flap valve and the apposition of sealing surfaces are typically evaluated by endoscopic visualization in which the examining endoscope is retroflexed to view the opening of the esophagus into the stomach. The shaft of the endoscope proximal to the retroflexed segment traverses the opening and thus the valve and sealing surfaces are viewed in the context of their contact with the shaft of the endoscope. By way of example, Hill and colleagues developed the following grading system to describe the appearance of the flap valve as thus viewed: Grade I, in which there is a prominent fold of tissue extending along the shaft of the endoscope and closely apposed to the endoscope through all phases of respiration; Grade II, in which the fold is less prominent and occasionally opening and closing around the endoscope during respiration; Grade III, in which a fold is present but is neither prominent nor in close contact with the endoscope; and Grade IV, in which there is no fold present and the opening is agape about the endoscope. Hill LD et al. 1996, Gastrointest Endosc 44:541-7. Following the conventions provided by this general scheme, it is evident that sealing surfaces can be classified as apposed in Grade I. Not evident under this scheme, but nonetheless possible, sealing surfaces can be apposed in any situation, regardless of the presence or absence of any fold of tissue, provided there is continuous contact between the entire circumference of the endoscope shaft and tissue at the junction between the esophagus and the stomach.

The three methods described below are based upon the endoscopic appearance of the gastroesophageal flap valve (Table 1). A first method is directed to the clinical situation where there is a sufficient flap present but the sealing surfaces are not apposed. The method involves bringing the sealing surface and the flap closer together to tighten an existing flap valve. A second method is directed to the clinical situation where there is not a sufficient flap present but the sealing surfaces are apposed. The method involves the creation of a flap when there is none present, and alternatively, augmentation of an existing flap that is simply not large enough to cover the opening of the esophagus into the stomach. A third method is directed to the clinical situation where there is neither a sufficient flap present nor apposition of sealing surfaces. The method involves creating or augmenting a flap and bringing the flap or sealing surfaces closer together.

**Table 1. Selection Criteria for Treating GERD Based upon Endoscopic Evaluation**

| ENDOSCOPIC EVALUATION | | TREATMENT | |
|---|---|---|---|
| Is a sufficient flap of tissue present? | Are the sealing surfaces apposed? | FIG. number | Rationale |
| Yes | Yes | 14 | No treatment required |
| Yes | No | 15, 16, 17 | Brings sealing surfaces closer together to tighten existing flap valve |
| No | Yes | 4, 5, 6, 18, 19 | Creates or augments flap |
| No | No | 20, 21, 22, 23, 24 | Creates or augments flap and brings sealing surfaces closer together |

The three above methods are performed at least in part with endoscopic visualization of stomach tissue for at least a part of one or more steps of the procedure. A preferred instrument for practicing the methods is disclosed in a separate section below. Other aspects of each of the three methods will now be discussed in more detail.

### Sufficient Flap Present but Sealing Surfaces Inadequately Apposed: Creation of a Bulge or Tightening of Existing Flap Valve.

To remedy the problem where there is a sufficient flap present but the sealing surfaces are inadequately apposed, as shown in FIGS. 14 and 15, inner surface 16 of stomach 10, optionally including at least one underlying layer of stomach wall 34, is engaged at two or more independent points 73 and 75. Points of engagement 73 and 75 are disposed near the opening of the esophagus into the stomach 36, and on the side of the opening of the esophagus into the stomach 36 opposite the existing flap portion 67 of gastroesophageal flap valve 70. The positions of points of engagement 73 and 75 as shown in FIG. 15 are not meant to be limiting. As shown in FIG. 15, the positions of the points of engagement 73 and 75 and of the flap portion 67 are related by their disposition on opposite sides of the opening of the esophagus into the stomach 36. Accordingly, in clinical practice the positions of points of engagement 73 and 75 depend on the position of the flap portion 67, so that positions of points of engagement 73 and 75 and flap portion 67 could differ from those illustrated in FIG. 15 by rotation about the opening of the esophagus into the stomach 36 by as much as 180°. Engagement points 73 and 75 are then moved toward each other in the direction of the arrows shown in FIG. 15, creating a tissue bulge or mound 72. This action can be readily accomplished by a manipulation that involves squeezing. Tissue bulge 72 so created displaces the sealing surface of valve seat 69 toward the sealing surface of flap portion 67. This manipulated stomach tissue is fixed by deployment of at least one tissue fixation device, in the manner previously described, at tissue fixation point 77 to retain the shape of the tissue bulge 72. The tissue bulge 72 thus established permanently displaces the sealing surface of valve seat 69 toward the sealing surface of existing flap portion 67, effectively reconstituting a competent flap valve.

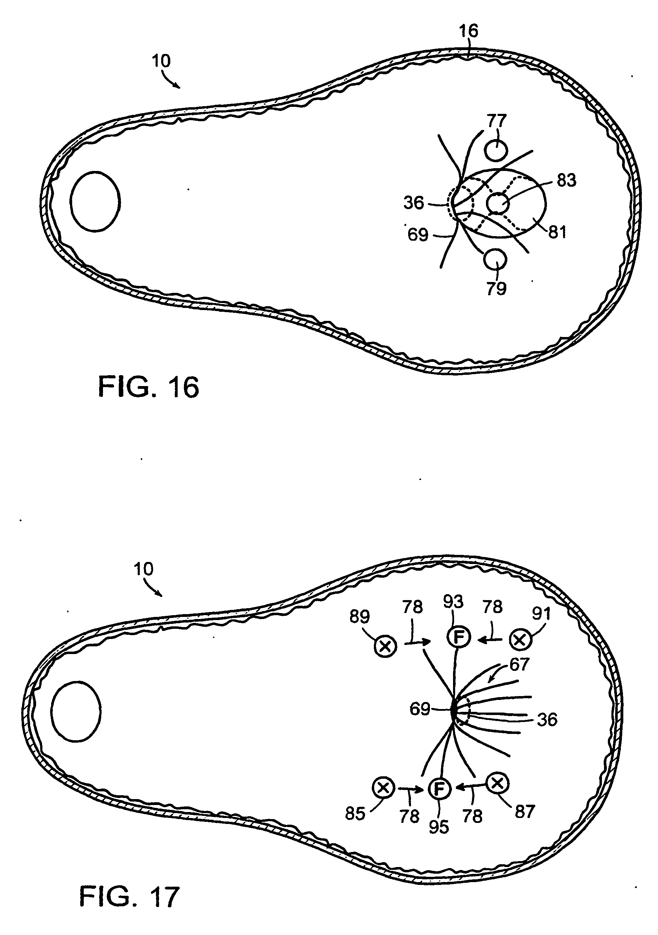

In another preferred embodiment of this method for remedying this problem, as shown in FIG. 16, stomach tissue is engaged at two points 77 and 79 near the opening of the esophagus into the stomach 36, on the same side of the opening of the esophagus into the stomach 36 as the existing flap portion 67 of gastroesophageal flap valve 70. The positions of points of engagement 77 and 79 as shown in FIG. 16 are not meant to be limiting. As shown in FIG. 16, the positions of the points of engagement 77 and 79 and of the flap portion 67 are related by their disposition on the same side of the opening of the esophagus into the stomach 36. Accordingly, in clinical practice the positions of points of engagement 77 and 79 depend on the position of the flap portion 67, so that positions of points of engagement 77 and 79 and flap portion 67 could differ from those illustrated in FIG. 16 by rotation about the opening of the esophagus into the stomach 36 by as much as 180°. Stomach tissue thus engaged at points 77 and 79 is manipulated to bring them into closer approximation. Such a manipulation creates a tissue bulge 81 that displaces the sealing surface provided by the flap portion 67 toward the sealing surface 69 on the opposite side of the opening of the esophagus into the stomach 36. Manipulated stomach tissue is fixed by deployment of at least one tissue fixation device at a tissue fixation point 83 to retain the shape of the tissue bulge 81. The tissue bulge 81 thus established permanently displaces the sealing surface provided by the existing flap portion 67 toward the sealing surface 69 on the opposite side of the opening of the esophagus into the stomach 36, effectively reconstituting a competent flap valve.

In yet another preferred embodiment of this method for remedying this problem, as shown in FIG. 17, stomach tissue is engaged at two or more pairs of independent points of tissue engagement, one pair defined by points 85 and 87, and another pair defined by points 89 and 91, near the opening of the esophagus into the stomach 36. The pairs of points are preferably disposed about the opening of the esophagus into the stomach 36 as follows: points 87 and 91 are on the same side as the flap portion 67, and points 85 and 89 are on the side of the opening of the esophagus into the stomach 36 opposite the flap portion 67. Accordingly, in clinical practice the positions of pairs of points of engagement depend on the position of the flap portion 67, so that positions of points of engagement 85, 87, 89, and 91 and flap portion 67 could differ from those illustrated in FIG. 17 by rotation about the opening of the esophagus into the stomach 36 by as much as 180°. Pair of points 85 and 87 may be engaged in one step of the method, and pair of points 89 and 91 may be engaged in a separate step of the method. Both points 85 and 87 and points 89 and 91 are independently manipulated in the direction of arrows 78 to bring points 85 and 87 into closer approximation, as well as to bring points 89 and 91 into closer approximation. At least two tissue fixation devices are deployed into stomach tissue at fixation points 93 and 95 in the manner previously discussed to retain the configuration achieved by the manipulation steps. Points 85 and 87 may be secured in one step of the method, and points 89 and 91 may be secured in a separate step of the method. The securing step in FIG. 17 may involve securing manipulated segments of tissue to each other (e.g., point 85 to point 87 and point 89 to point 91) or securing each independent manipulated segment to an unmanipulated portion. Unlike the embodiments above, this embodiment of this method need not necessarily result in the creation of a tissue bulge. However, this embodiment of this method also brings into apposition the sealing surfaces provided by the existing sufficient flap portion 67 and the sealing surface 69 on the opposite side of the opening of the esophagus into the stomach 36, effectively reconstituting a competent flap valve.

### Sufficient Flap Not Present but Sealing Surfaces Adequately Apposed: Creation or Augmention of a Flap.

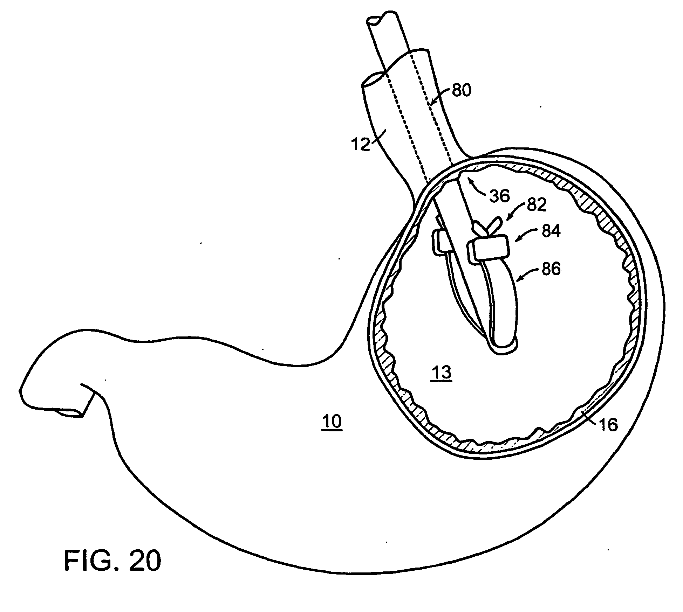

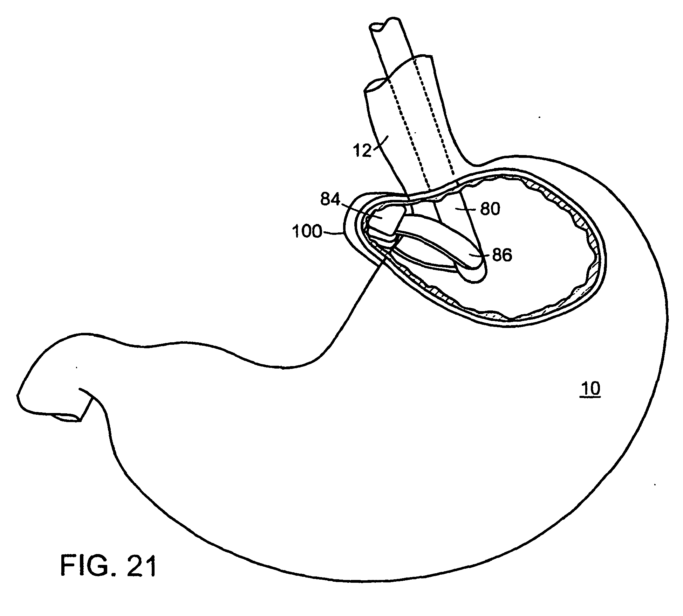

To remedy a situation where a sufficient flap is not present but the sealing surfaces are adequately apposed, the method already described with respect to FIGS. 20 and 21 is used. Stomach lining tissue 16, optionally including at least one underlying layer of stomach wall 34, is engaged at each point 37 and 39 near the opening of the esophagus into the stomach 36. Each point 37 and 39 must be positioned relative to opening of the esophagus into the stomach 36 so that subsequent manipulation and fixation steps result in a flap of stomach tissue suitably located and of sufficient area to cover at least a significant portion of opening of the esophagus into the stomach 36. Stomach tissue engaged at each point 37 and 39 is manipulated in the direction of arrows 38, i.e., in a direction toward opening of the esophagus into the stomach 36. The manipulations of the two points 37 and 39 shown in FIG. 4 may be accomplished sequentially or simultaneously, with the result of being the formation or augmentation of a substantially rectangular flap 40, as shown in FIG. 5. Stomach tissue is then secured to flap 40 at two fixation points 35 in the manner described previously, to maintain the substantially rectangular shape of the flap 40 . The size and tightness of the flap 46 overlying the opening of the esophagus into the stomach 36 will vary based on the location of fixation points 35.

In an alternative embodiment of this method shown in FIG. 6, the engagement and manipulation of a single point 41 in the direction of and across the opening of the esophagus into the stomach 36 results in the formation or augmentation of a substantially triangular flap 50. The substantially triangular flap 50 is secured by a single tissue fixation device to stomach tissue at tissue fixation point 51, in the manner previously described.

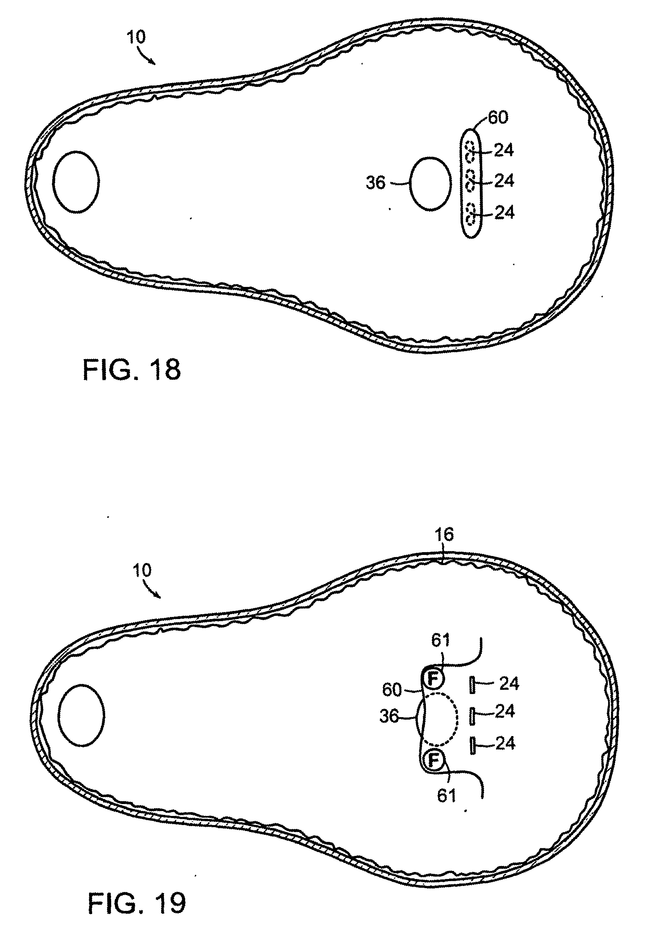

FIGS. 18-20 depict yet another alternative embodiment of this method for remedying the problem where there is not a sufficient flap but the sealing surfaces are adequately apposed. Lining tissue 16 is engaged at points 37 and 39 near and on one side of the opening of the esophagus into the stomach 36 and is invaginated to form a rectangular invaginated flap 60 (FIGS. 18 and 19). In the view of FIG. 18, the flap 60 is coming up out of the plane of the paper. After the engagement and manipulation steps shown in FIGS. 18 and 20, substantially rectangular flap 60 is fixed at its base with one or more tissue fixation devices 24. In a second step, depicted in FIG. 19, the free margin of the resulting flap 60 is pulled toward the opposite side of the opening of the esophagus into the stomach, positioned to cover the opening of the esophagus into the stomach 36, and fixed to stomach tissue in this position at one or more points 61 opposite the opening of the esophagus into the stomach 36 from the fixed base of the flap 60. Two tissue fixation devices each pass through at least two layers of stomach wall: the layer or layers forming the free margin of the flap 60 and at least the lining 16 of the stomach wall near opening of the esophagus into the stomach 36. The size and tightness of the flap depend on the location of points of fixation 61 relative to the base of the flap 60 and the position of the opening of the esophagus into the stomach 36.

### Sufficient Flap Not Present and Sealing Surfaces Indequately Apposed: Creation or Augmentation of a Flap Combined with Bringing Sealing Surfaces Closer Together.

To remedy a situation where a sufficient flap of tissue is not already present and the sealing surfaces are not apposed, the stomach is plicated by forming and securing a pair of evaginations by a technique discussed below with respect to FIGS. 20-23.

As shown in FIG. 20, an endoscopic tissue engaging and tissue securing device 80 is introduced into the lumen 13 of the stomach 10 through lumen 11 of the esophagus 12. The distal end of the tissue engaging and securing device 80 includes a pair of tissue engaging elements 82 and a pair of tissue securing device elements 84 each disposed on a rotatably positionable arm 86. The two tissue securing device elements 84 can be two aspects of a single device, e.g., one element can be the anvil for the other. Alternatively, the two tissue securing device elements 84 can be two independent tissue securing devices unto themselves, e.g., two one-sided staplers. Motions of the two movable arms 86 can be dependently linked or can be independent of one another. The operator selects two sites on the lining 16 of the stomach and adjacent to opening of the esophagus into the stomach 36 to be engaged by the two tissue engaging elements 82.

FIG. 21 depicts how, having once engaged tissue at both sites, the operator then causes the arms 86 of the combination endoscopic tissue engaging device/tissue securing device 80 to swing, thereby creating a pair of evaginations 100 straddling the GEJ or distal esophagus. The two evaginations 100 are then caused to come into apposition, and the two tissue securing device elements 84 deploy at least one tissue fixation device to affix one evaginated portion 100 to the other.

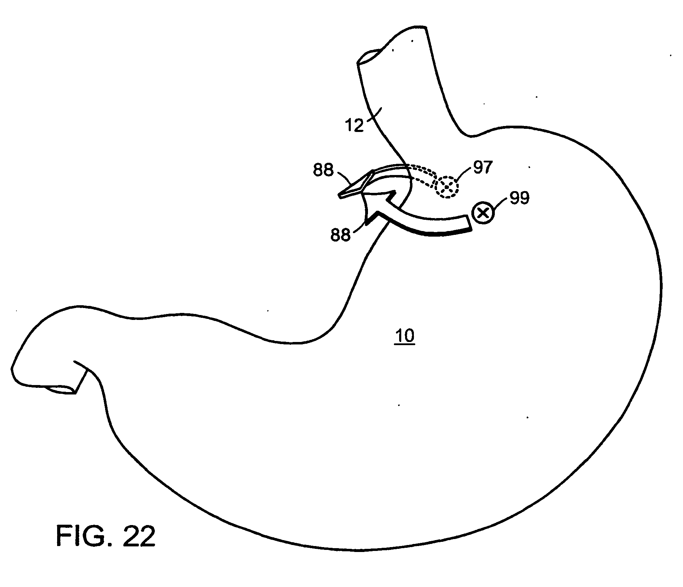

FIG. 22 depicts an exterior view of stomach 10 (without the combination endoscopic tissue engaging device/tissue securing device 80) showing external counterparts 97 and 99 to the sites of tissue engagement (which are within the stomach) and the intended direction of tissue manipulation, as shown by arrows 88.

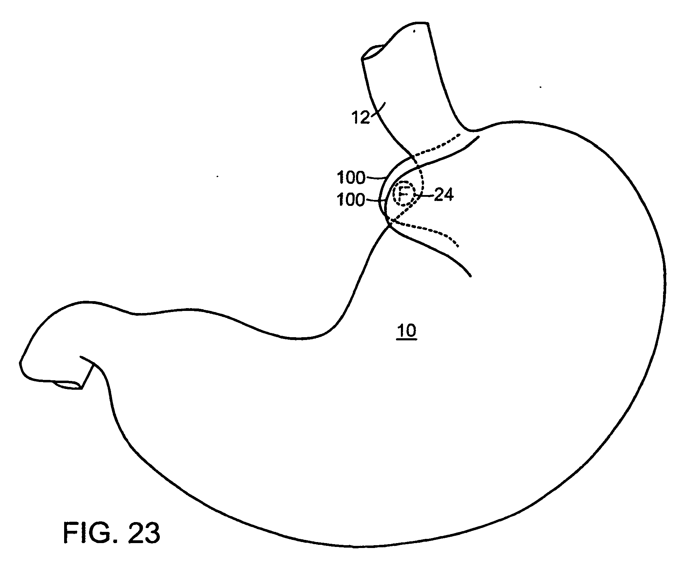

As discussed above, fixation is accomplished by placement from the lumen 13 of the stomach 10 of at least one tissue fixation device 24 through at least one full-thickness layer of stomach wall. FIG. 23 depicts an exterior view of stomach 10 following deployment of tissue fixation device 24 connecting apposing surfaces of evaginations 100 to create a partial gastric wrap. Tissue fixation device 24 is drawn in broken lines to indicate it is not visible from the outside of the stomach.

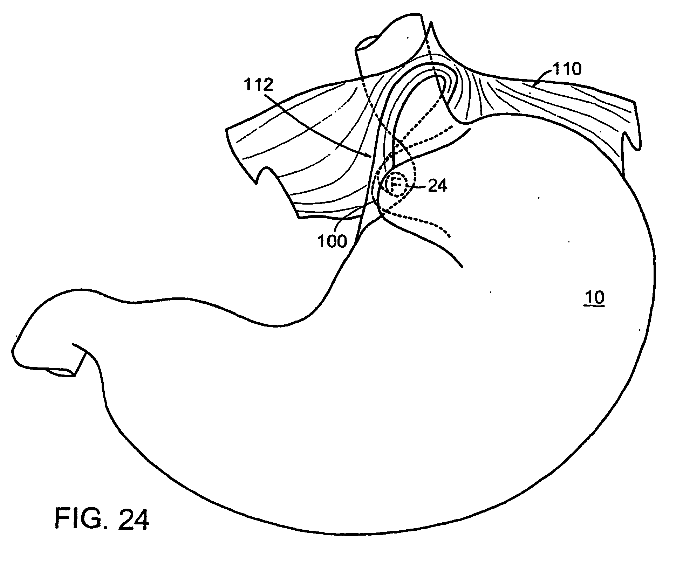

In one embodiment of this method, as depicted in FIGS. 20-22 and 24 and described in greater detail below, at least one of evaginations 100 is brought into contact with and secured to a tissue structure extrinsic to stomach 10. The tissue structure extrinsic to stomach 10 is preferably an aspect of diaphragm 110. In one particularly preferred embodiment, an aspect of the diaphragm 110 is interposed between evaginations 100 and the mutual fixation of evaginations 100 with fixation device 24 simultaneously fixes the interposed aspect of diaphragm 110 to evaginations 100. Securing of a portion of stomach 10 near the GEJ to a relatively immobile structure extrinsic to stomach 10 creates or assures an effective barrier to reflux. Hill LD et al. (1990) Gastroenterol Clin North Am 19:745-75.

### C. Endoscopic Methods for Repairing a Hiatus Hernia

This invention can be used in endoscopic methods for repairing a hiatus hernia. The methods include engaging an aspect of the stomach from within, reducing the hernia (i.e., manipulating a portion of the stomach so engaged to reposition the herniated portion beneath the diaphragm, manipulating a portion of the stomach so engaged to bring it into contact with an aspect of a tissue structure extrinsic to the stomach, and securing a portion of a stomach to an aspect of a tissue structure extrinsic to the stomach.

According to a preferred aspect the tissue extrinsic to the stomach is an aspect of the diaphragm 110. In a most preferred aspect the tissue extrinsic to the stomach is the median arcuate ligament. In other preferred embodiments the tissue extrinsic to the stomach can involve tissue of the right crus, left crus, preaortic fascia, hepatogastric ligament, lesser omentum, or greater omentum.

A preferred method involves evagination, much like the method depicted in FIGS. 20-23, with the additional feature of engaging and fixing an aspect of a tissue structure extrinsic to the stomach between evaginated portions 100.

Preferably, as shown in FIGS. 20-22 and FIG. 24, although not necessarily, an aspect of the diaphragm 110 is sandwiched and secured by tissue fixation device 24 between the two evaginations 100 of the stomach wall. The preferred aspect of diaphragm 110 is a portion of the median arcuate ligament 112. This method achieves the combined effects of anchoring the stomach 10 to the diaphragm 110, creating a flap element of a flap valve at the opening of the esophagus into the stomach 36, and bringing the sealing surfaces closer together, i.e., shifting the tissue at the base of the flap in the direction of the opening of the opening of the esophagus into the stomach 36.

An advantage of involving tissue extrinsic to the stomach is the potential to limit the freedom of movement of at least the secured portion of the stomach relative to some other organ or tissue. The importance of this limitation of movement is well recognized in the surgical treatment of GERD. Hill LD 1989, J Thorac Cardiovasc Surg 98:1-10. The classic Hill gastropexy, an open procedure, includes anchoring the GEJ to the median arcuate ligament of the diaphragm, thereby eliminating or at least reducing the mobility of a sliding hiatus hernia.

Endoscopic visualization may be used for all or any part of the method.

In a preferred embodiment, the at least one tissue fixation device 24 makes a through-and-through penetration of the sequential full thicknesses of stomach 10, entrapped aspect of diaphragm 110, and stomach 10. In another embodiment, the at least one tissue fixation device 24 makes both a through-and-through penetration of the full thicknesses of one face of the stomach 10 and entrapped aspect of diaphragm 110, and a partial penetration of the opposing face of stomach 10. Fixation can alternatively be effected by (1) applying at least one tissue fixation device 24 through the full thickness of one face of the stomach 10 into at least a partial thickness of the entrapped aspect of the diaphragm 110, and (2) applying at least one tissue fixation device 101 through the full thickness of a second face of the stomach 10 into at least a partial thickness of the entrapped aspect of the diaphragm 110. Similarly, combinations of full- and partial-thickness bilateral fixations can be employed.

According to another embodiment of this method, stomach 10 and an aspect of the extrinsic tissue structure 110 are brought into apposition through engagement and invagination of a portion of the inner aspect of stomach 10. Stomach 10 and extrinsic structure 110 are then fixed together by the application from the lumen 13 of the stomach of at least one tissue fixation device 24 to penetrate both the stomach and at least a partial thickness of the extrinsic tissue structure.

In yet another embodiment of this method, stomach 10 and extrinsic tissue structure 110 may already be naturally in desired apposition. In such applications there may be no need for the engaging and manipulating steps. The stomach and extrinsic structure are then fixed together by the application from lumen 13 of stomach 10 of at least one tissue fixation device 24 to penetrate both the stomach and at least a partial thickness of the extrinsic tissue structure.

### D. Instruments and Devices for Endoscopically Reconfiguring Tissue Within a Hollow Organ.

Instruments useful for reconfiguring tissue within a hollow organ will be discussed. Such instruments may incorporate at least two of the following aspects: tissue engagement device; tissue manipulation device; tissue securing device; and viewing endoscope. In a preferred embodiment, described below, there is a single instrument combining a tissue engaging device, tissue manipulation device, and tissue fixation device. A unique feature of this combination instrument is its ability to manipulate two or more points of tissue in any desired direction in three dimensional space.

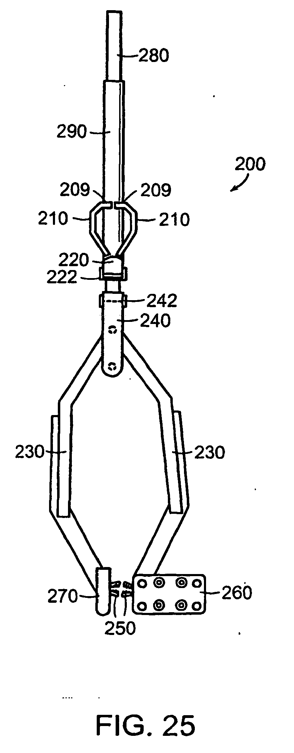

An example of a preferred combination instrument 200 incorporating a tissue engagement device and a tissue manipulation device will now be discussed with reference to FIGS. 25, 26, 30, and 32-36.

Instrument 200 includes inner tube 280, concentric outer tube 290, a pair of opposable grasper arms 210, grasper arms yoke 220, a pair of independent small graspers 250, articulable stapler arms 230, stapler arms yoke 240, stapler cartridge 260 and stapler anvil 270. Instrument 200 may be constructed so that it can be sterilized by any method known in the art, e.g., steam autoclaving, gamma irradiation, and gas sterilization. Grasper arms 210 are attached to grasper yoke 220 which is in turn attached by articulable joint 222 to outer tube 290. Grasper arms 210 are thus able to open and close in order to grasp, as well as to pivot about 180° as a unit relative to the long axis of the instrument. A pair of torsion springs 216 cause the grasper arms 210 to tend to assume an open position in which ends 209 thereof are spaced, and the grasper yoke 220 to tend to assume an off-axis position. Stapler arms 230 are attached to stapler yoke 240 which is in turn attached by articulable joint 242 to inner tube 280. Stapler arms 230 are thus able to open and close and to pivot about 180° as a unit relative to the long axis of the instrument. A pair of torsion springs 236 cause the stapler arms 230 to tend to assume an open position in which cartridge 260 and anvil 270 are spaced from one another, and the stapler yoke assembly 240 to tend to assume an on-axis position. The long axis of the instrument is defined by the concentric axes of inner tube 280 and outer tube 290. Tubes 280 and 290 are constructed so that inner tube 280 can slide and rotate within outer tube 290, thus permitting grasper yoke 220 and stapler yoke 240 to move in relation to each other along and about the long axis of the instrument. Stapler cartridge 260 and stapler anvil 270 are disposed near the spaced, distal ends of respective stapler arms 230. Cartridge 260 and anvil 270 can be specially structured or structured according to conventional designs which are well known in the art.

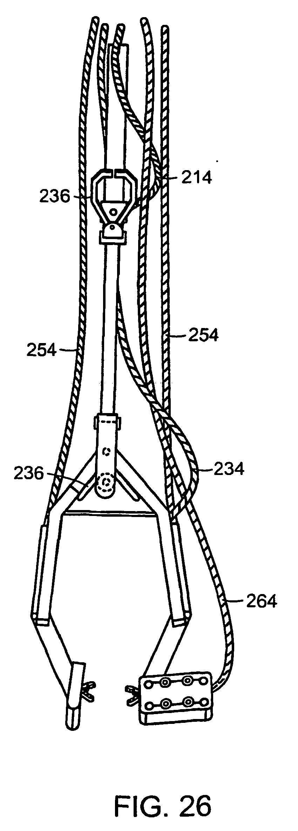

A small grasper 250 is disposed at the end of each stapler arm 230. As illustrated in FIGS. 25 and 26, each small grasper 250 includes two opposed, toothed jaws pivotally mounted at one end. Small graspers 250 are constructed and activated by a small grasper cable assembly 254 in the same manner as commercially available endoscopic graspers, forceps, biopsy forceps that are well known in the art.

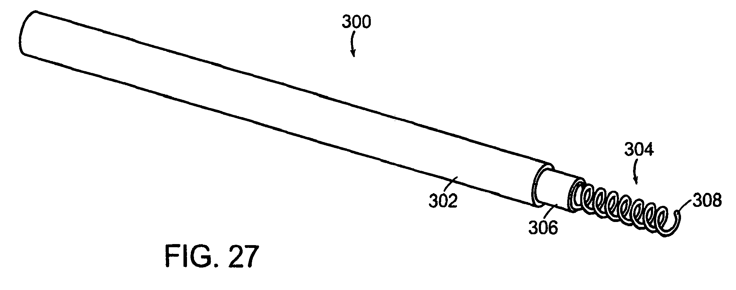

In a more preferred embodiment, either or both of small graspers 250 are substituted with a helical tissue engagement device 300 as depicted in FIG. 27. Helical device 300 has a shape much like a corkscrew and includes a distal effector end operably connected by a shaft to a proximal controlling end which remains outside the subject when in use. As shown in FIG. 27, the distal end of tissue engaging device 300 includes a generally helical spiral 304 having sharpened end 308 and being attached to a shaft 306 which is at least somewhat flexible along its length but sufficiently rigid to transmit a torque to spiral 304 to allow spiral 304 to be screwed into and out of tissue contacted by the sharpened end 308 of spiral 304. Spiral 304 having sharpened end 308 is structured to engage tissue when turned in one direction and to release tissue when turned in the opposite direction. Spiral 304 will typically be made of titanium, stainless steel, or like material suitable for surgical instrumentation with a wire diameter of about 0.381-1.02mm (0.015"-0.040"). In a preferred embodiment, spiral 304 wire diameter is 0.635mm (0.025") Example dimensions for spiral 304 include radial outside diameter 2.03-6.35mm (0.080" - 0.250"), and, in a preferred embodiment, 3.05mm (0.120"). Corkscrew-type tissue engaging device 300 is advanced through a working channel of an endoscopic instrument. Alternatively, corkscrew-type tissue engaging device 300 is slidably disposed within an overtube 302 so that the operator can cause sharpened end 308 of spiral 304 to protrude beyond and retract within the distal end of the overtube 302 by a desired amount. Overtube 302 may be made of stainless steel, extruded polymer with embedded stainless steel braid, polyethylene, polypropylene, polyimide, TEFLON (RTM), or similar suitable biocompatible material. In another alternative, corkscrew-type tissue engaging device 300 is advanced through a working channel of the combination instrument 200. Such a working channel is a hollow tube suitable to accommodate a remotely operated surgical tool such as corkscrew-type tissue engaging device 300,a needle, a grasper, a biopsy device, a brush, an electrocautery electrode, and the like.

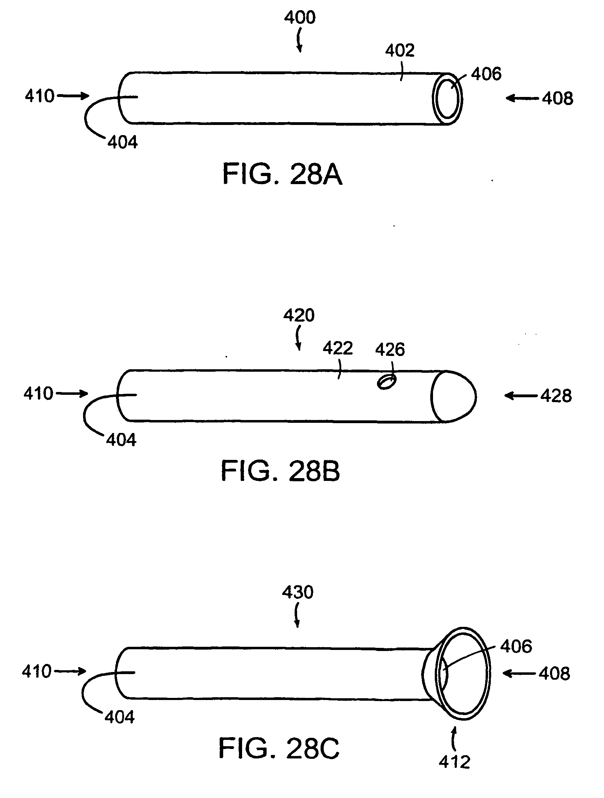

In yet another embodiment, either or both of graspers 250 is substituted with suction devices as depicted in FIG. 28. "Suction" as used herein is equivalent to vacuum or reduced pressure, relative to ambient atmospheric pressure. In its simplest embodiment, shown in FIG. 28, the suction-based tissue engaging device 400 is an open-ended tube 402 with aperture 406 at its distal end 408, tube 402 constructed with sufficient axial rigidity to resist collapse under the force of an effective vacuum existent within its lumen 404 when applied end-on to tissue at distal end 408. In another embodiment shown in FIG. 28 the suction-based tissue engaging device 420 is a blind-ended tube 422 with at least one aperture 426 in a side wall near distal end 428, tube 422 having sufficient axial rigidity to resist collapse under the force of an effective vacuum existent within its lumen 404 when applied side-on to tissue. End or side apertures 406 or 426 can include a flange 412. FIG. 28 illustrates one such embodiment 430 in which aperture 406 at distal end 408 of open-ended tube 402 opens into flange 412 in fluid communication with lumen 404. Flange 412 can take the shape of a cone, cup, portion of a sphere, or otherwise smoothly concave surface. The source of vacuum or reduced pressure can be provided by operative connection at proximal end 410 to any means well known in the art, provided the vacuum supplied is effective for engaging tissue and suitable for the purposes of the invention. Such means include, without limitation, commercially available vacuum pumps, "wall suction" available in any hospital operating room and in many medical or surgical procedure rooms and many patient rooms at a hospital, side-arm aspirator, and the like. Reduced pressures suitable for the purposes of the invention typically fall between 10 and about 560 mm Hg but may vary with aperture size and shape.

For the purposes of the description which follows, graspers 250 are understood to be non-limiting, i.e., corkscrew-like retractors 300 or suction devices 400 can be used to similar effect.

As shown in FIG. 26, cable assembly 214 is coupled to grasper arms 210 and, together with grasper arms torsion springs 216 causes grasper arms 210 to open and close. Tensioning grasper arms cable assembly 214 counteracts grasper arms torsion springs 216 to cause grasper arms 210 to close; relaxing grasper arms cable assembly 214 permit grasper arms torsion springs 216 to cause the grasper arms 210 to open. ; Similarly, cable assembly 234 is coupled to stapler arms 230, and together with stapler arms torsion springs 236 cause to permit stapler arms 230 to open and close. Tensioning stapler arms cable assembly 234 counteracts stapler arm torsion springs 236 to cause stapler arms 230 to close; relaxing stapler arms cable assembly 234 permits stapler arms torsion springs 236 to cause stapler arms 230 to open.

Stapler cartridge 260 is disposed on the end of one stapler arm 230 and is activated by cable assembly 264 to deploy at least one staple into tissue. Stapler anvil 270 disposed on the distal end of the other stapler arm 230 and stapler cartridge 260 are brought into apposition by tensioning stapler arms cable assembly 234.

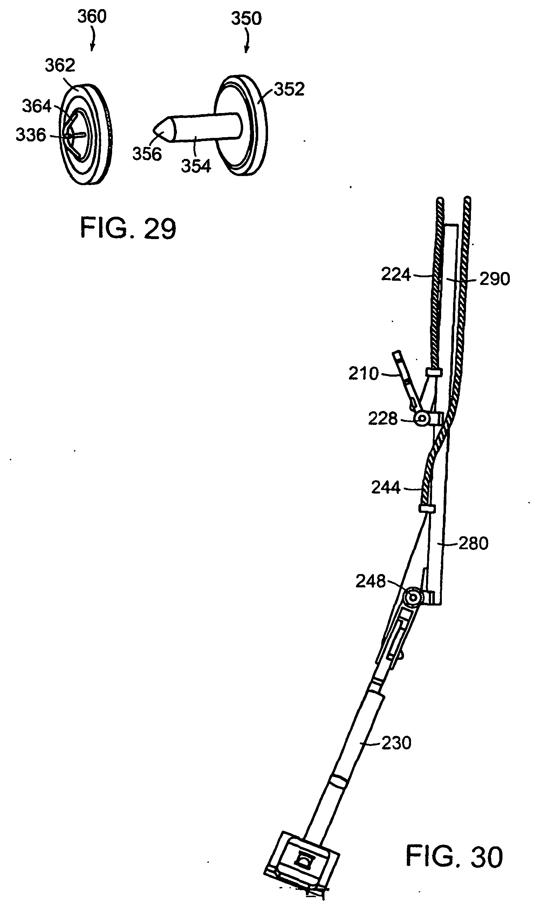

In an alternative embodiment, stapler cartridge 260 and stapler anvil 270 are substituted with corresponding elements structured to deliver at least one two-part fastener as described above. An example of a preferred embodiment of a two-part fastener is shown in FIG. 29. The fastener includes a first part 350 and a second part 360. First part 350 includes a head 352 and a post 354 having a conical end which tapers to a point 356 capable of piercing tissue. Second part 360 is an annular retainer 362 structured to engage post 354 with retainer slotted flange 364 when point 356 is advanced through retainer aperture 366. Slotted flange 364 includes a plurality of rigid radially extending flaps which also extend axially away from head 352 on one side of aperture 366. Flaps of slotted flange 364 allow post 354 to be inserted through aperture 366 from the other side of aperture 366, but prevent withdrawal of post 354 once inserted. Flaps of slotted flange 364 bend radially outwardly to accommodate post 354, the diameter of which otherwise exceeds the aperture 366, thus causing slotted flange 364 to engage and retain post 354. The length of post 354 is sufficient to penetrate through the desired amount or depth of tissue and to permit engagement by retainer 362 for the application for which it is used. Typically, such length will be ca. 6.35 mm (0.25 inches). In a preferred embodiment the greatest outside diameter of head 352 or retainer 362 is ca. 6.35mm (0.250 inches). Post 354 can be grooved or threaded, e.g., with an 0-80 thread to provide a more secure engagement with slotted flange 364. Parts 350 and 360 preferably are made of titanium, stainless steel, biocompatible polymer, or a combination of such materials. For the purposes of the description which follows, stapler cartridge 260 and stapler anvil 270 are understood to be non-limiting, i.e., elements structured to deliver at least one two-part fastener could be used to similar effect.

As shown in FIG. 30; grasper arms yoke cable assembly 224 and grasper arms yoke torsion spring 228 are coupled to grasper arms yoke 220. Grasper arms yoke 220 and grasper arms 210 pivot about articulable joint 222. Tensioning of grasper arms yoke cable assembly 224 counteracts the grasper arms yoke torsion spring 228 to cause the grasper arms yoke 220 to pivot so that the free ends 209 of grasper arms 210 pivot away from stapler arms yoke 240; relaxing of grasper arms yoke cable assembly 224 permits grasper arms yoke torsion spring 228 to cause the grasper arms yoke 220 to pivot so that the free ends 209 of grasper arms 210 pivot toward the stapler arms yoke 240.

Similarly, stapler arms yoke cable assembly 244 and stapler arms yoke torsion spring 248 are coupled to stapler arms yoke 240. Yoke 240 pivots about an articulable joint 242. Tensioning of stapler arms yoke cable assembly 244 counteracts stapler arms yoke torsion spring 248 to cause the stapler arms yoke 240 to pivot so that the free ends of stapler arms 230 pivot toward grasper arms yoke 220; relaxing of stapler arms yoke cable assembly 244 permits stapler arms yoke torsion spring 248 to cause stapler arms yoke 240 to pivot so that the free ends of grasper arms 230 pivot away from grasper arms yoke 220.

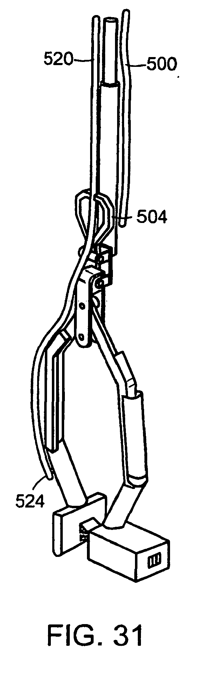

An optional feature of instrument 200 shown in FIG. 31 is the inclusion of at least one of pressure monitoring tubes 500 and 520. Tube 500 includes at least one opening 506 positioned in the vicinity of the grasper arms yoke 220 and permits measurement of tissue pressure at the GEJ when instrument 200 is advanced into position as previously described. The proximal end of tube 500 is operably connected to a manometer outside the subject. Tube 520 includes at least one opening 506 positioned anywhere along stapler arms 230 and permits measurement of yield pressure when instrument 200 is advanced into position as previously described. The proximal end of tube 520 is operably connected to a manometer outside the subject. Tubes 500 and 520 are preferably made of a biocompatible polymer and are structured to have an inside diameter of at least ca. 0.508mm (0.020 inches). Tube 500 terminates as a blind-ended tube at its distal end 504 and has an opening 506 in its side wall near distal end 504. Tube 520 may also terminate as a blind-ended tube at its distal end 504 and has an opening 506 in its side wall near distal end 524. Alternatively, tube 520 may terminate as an open-ended tube at distal end 524.

Measurement of the tissue pressure at the GEJ, the yield pressure, or both pressures assists the operator in determining where to engage, manipulate, and/or secure tissue to greatest advantage. Pressure measurements can be taken at any point throughout the procedure. For example, an operator performing any of the described methods for treating GERD can take a baseline measurement of at least one of these pressures, engage and manipulate tissue, take another measurement, disengage tissue, and then repeat at least the steps of engaging, manipulating, and measuring, until a desired pressure is obtained. Likewise, an operator performing any of the described methods for treating GERD can take a baseline measurement of at least one of these pressures, engage and manipulate tissue, take another measurement, disengage tissue, and then repeat at least the steps of engaging, manipulating, and measuring, to determine an optimal location of engagement, an optimal manipulation, and/or an optimal point of fixation.

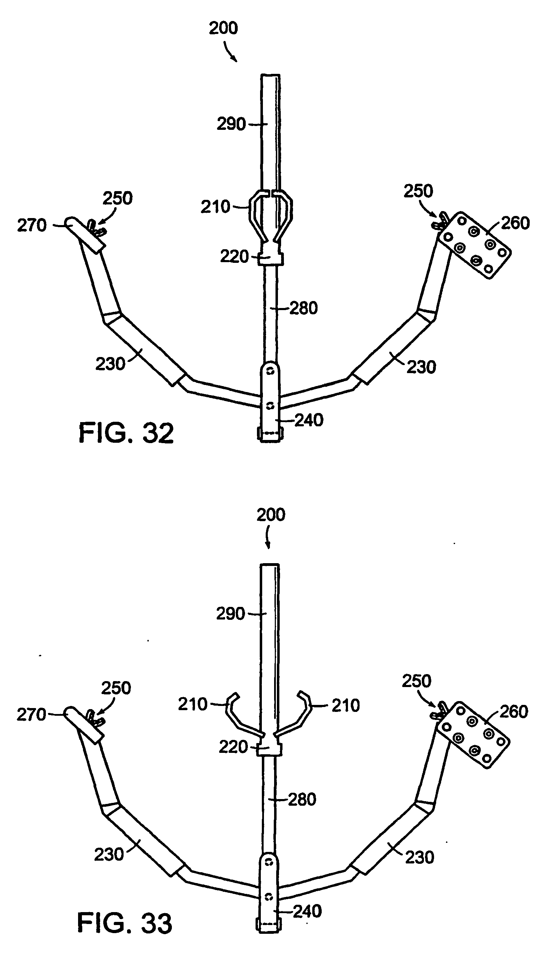

When instrument 200 is used for the above indicated methods, the distal end of instrument 200, including stapler arms 230, stapler arms yoke 240, grasper arms yoke 220, and grasper arms 210, is introduced into stomach 10 of a subject via a conduit which may be esophagus 12 or a gastrostomy. During the introduction of the instrument into the stomach, the instrument is positioned as shown in FIG. 25. When positioned as shown in FIG. 25, the entire distal end of instrument 200 is structured to pass through a hole less than about 25.4mm (one inch) in diameter, and more preferably, through a hole no more than 2.0 cm in diameter. Next, stapler arms yoke 240 is rotated by tensioning stapler arms yoke cable assembly 244, stapler arms 230 are opened by relaxing stapler arms cable assembly 234, and inner tube 280 is advanced within outer tube 290. The instrument thus assumes the configuration shown in FIG. 32.

The two small graspers 250 engage tissue at two independent points. Tissue so engaged can be manipulated simply by tensioning stapler arms cable assembly 234, to bring the two small graspers 250 into closer proximity. Such manipulation can be used to create a mound of tissue by effectively squeezing tissue interposed between the two small graspers 250. Such an operation may be useful in bringing sealing surfaces closer together to tighten an existing flap valve, such as in FIGS. 15 and 17. In FIG. 15, small graspers 250 engage tissue at points 73 and 75 and stapler arms 230 are brought into closer approximation by tensioning stapler arms cable assembly 234 to create a mound of tissue 72 adjacent to the existing flap valve 70. At least one staple is deployed by stapler cartridge 260 into the tissue at location 77 to stabilize the tissue reconfiguration. Similarly in FIG. 17, small graspers 250 engage tissue at first pair of points 89 and 91 (or 85 and 87) and stapler arms 230 are brought into closer approximation by tensioning stapler arms cable assembly 234 to bring points 89 and 91 (or 85 and 87) into closer approximation. At least one staple is deployed by cartridge 260 into the tissue at respective location 93 (or 95) to stabilize the tissue reconfiguration. These steps are then repeated with respect to the other pair of points.

The two small graspers 250 can evaginate tissue by sliding the stapler arms yoke 240 in the direction of the desired evagination, such as shown in FIG. 17. Grasper arms 86 correspond to stapler arms 230; associated small graspers 82 to small graspers 250; and stapler elements 84 to stapler cartridge 260 and stapler anvil 270.

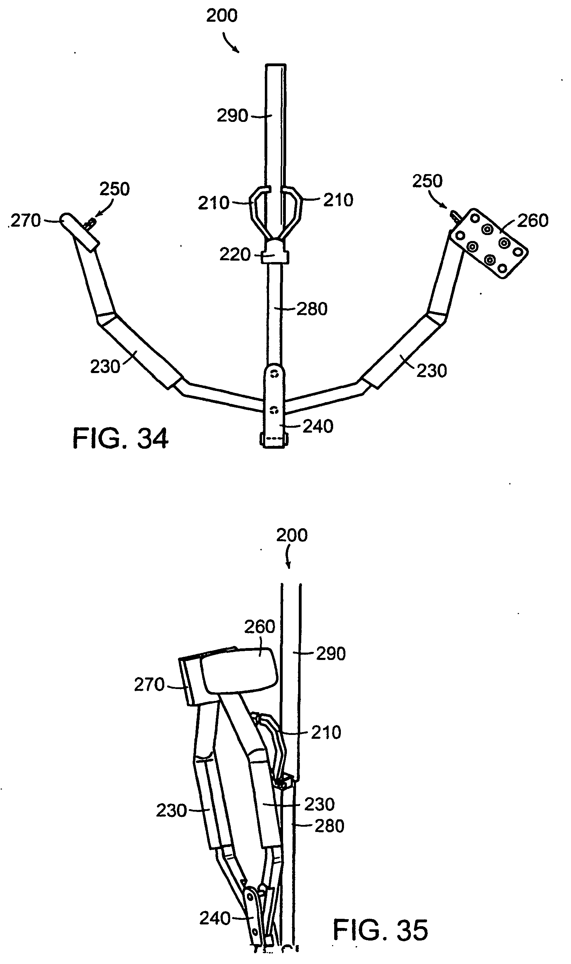

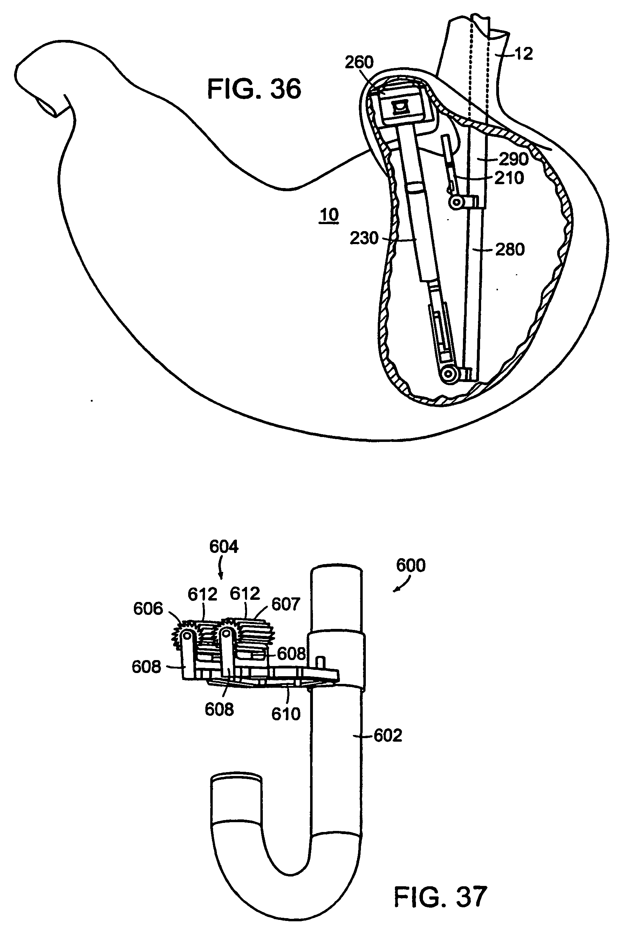

Small graspers 250 also may be used to advantage in combination with grasper arms 210. After introduction of the distal end of instrument 200 into the lumen of the stomach and assumption of configuration shown in FIG. 32 as above, FIG. 33 shows small graspers 250 and grasper arms 210 are opened and ready to be brought into contact with tissue. Following tissue contact, FIG. 34 shows grasper arms 210 closed to engage tissue near opening of esophagus into stomach 36, while small graspers 250 are closed to engage tissue at points that will be moved to create a plication, as shown in FIG. 22. As shown in FIG. 35, tissue so engaged can be manipulated by any combination of pivoting grasper arms 210, pivoting stapler arms 230, and positioning grasper arms yoke 220 relative to stapler arms yoke 240. In a preferred embodiment, stapler arms 230 are closed and stapler arms yoke 240 is pivoted as required to bring tissue together around the distal esophagus, creating a plication, as shown in FIG. 36.

Another embodiment of a tissue engaging and manipulating device 600 is illustrated with respect to FIG. 37. Device 600 includes a conventional endoscope 602 onto which or in conjunction with which a roller assembly 604 is mounted. Roller assembly 604 includes a pair of rollers 606 and 607 which are each independently journalled for free rotation on a pair of support arms 608. Support arms 608 in turn are fixed to support structure 610 which is coupled to endoscope 602. Each roller 606 and 607 includes teeth 612. Teeth 612 of one roller 606 preferably interengage teeth 612 of the other roller 607. However, in an alternative embodiment, the ends of teeth 612 on each roller 606 and 607 could be spaced a very small distance from one another. Preferably, teeth 612 extend from rollers 606 and 607 at an angle with respect to a radius of rollers 606. However, teeth 612 could extend radially. Rollers 606 and 607 rotate in opposite directions from one another. In other words, while roller 606 would rotate in a clockwise direction as shown in FIG. 37, roller 607 would rotate in a counter-clockwise direction, as shown in FIG. 37. In this way, tissue engaged by rollers 606 and 607 would be captured and drawn between rollers 606 and 607 to form a flap, bulge, mound or the like. Preferably, the direction of rotation of rollers 606 and 607 could be reversed to release tissue once fixation of the tissue had been obtained. Alternatively, support structure 610 can be structured so as to permit rollers 606 and 607 to disengage by swinging apart from one another. At least one of rollers 606 and 607 is driven by an externally disposed hand-operated mechanism or servo motor (not shown) which is coupled to each driven roller 606 and/or 607 by a suitable cable (not shown).

In operation, the combined endoscope and device 600 is deployed endoscopically into a subject's stomach. Utilizing viewing endoscope 602, device 604 is positioned to engage tissue at a desirable location within the stomach or other organ. Once placed in the desired location, the hand-operated mechanism or servo motor (not shown) is activated to rotate rollers 606 and 607 to engage and manipulate the tissue to a desired size and shape. Thereafter, rotation of rollers 606 and 607 is discontinued. A suitable fixation device (not shown) is deployed endoscopically to fix the resulting reconfigured tissue in its reconfigured shape. The tissue fixation device utilized is one of those previously described with respect to this invention, including a conventional stapler. Upon completion of the fixation step, the directions of rotation of rollers 606 and 607 are reversed to release the tissue from rollers 606 and 607. Thereafter, device 600 may be moved to a different location within the stomach or other organ and the steps described above may be repeated.

Since the device of the present invention according to claims 1 is novel and it is intended to be used in treating a human subject, it is important that the physician operator be instructed to use the device mechanisms and methods disclosed herein. The training of the device and method may be accomplished on a cadaver or a human model, or it may be accomplished at the bedside of a patient.

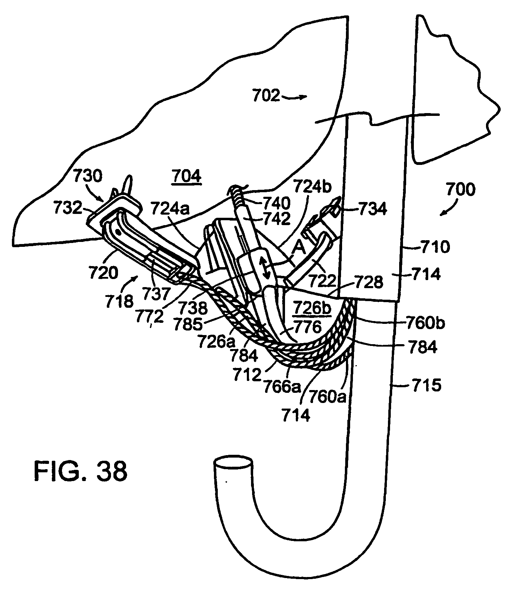

Referring to Fig. 38, an instrument 700 for reconfiguring stomach tissue, e.g., stomach tissue in the vicinity of the gastroesophageal junction (GEJ) 702, such as tissue 704 of the lesser curvature of the stomach, is shown. The GEJ is the region of transition from the esophagus to the stomach. The lesser curvature of the stomach is a portion of the stomach located beyond the GEJ. Instrument 700 includes an elongated shaft 710 dimensioned to permit transoral access to the stomach, and a tissue manipulator 712 for manipulating stomach tissue. Positioned within a lumen 714 defined by shaft 710 is a standard GI endoscope 7I5 providing visual guidance of the reconfiguring procedure. Instrument 700 is particularly adapted for treating GERD. Using instrument 700, as described below, a bulge, plication or tissue wrap is formed in the vicinity of gastroesophageal junction 702 to reduce reflux of stomach fluids into the esophagus.

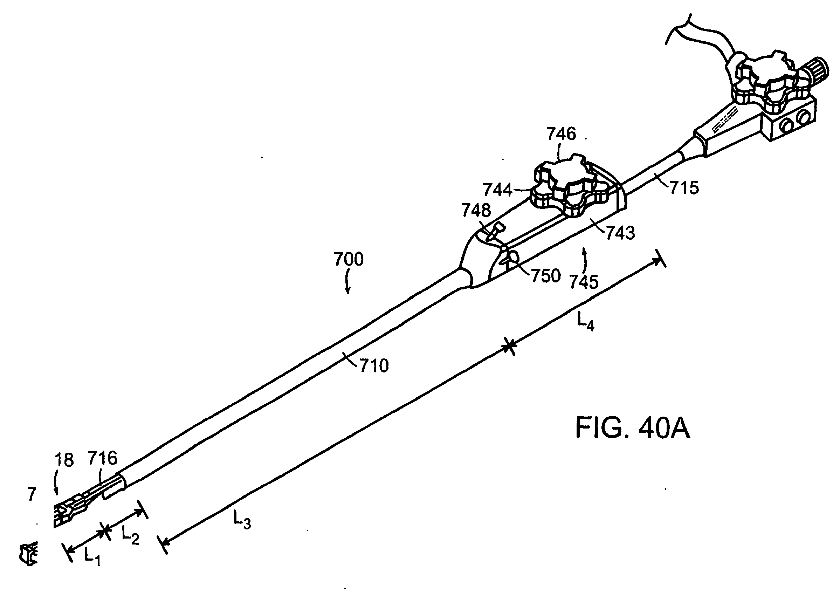

Tissue manipulator 712 has an elongated cable assembly 716 housed within lumen 714 of shaft 710, and a distal end effector 718 actuated to perform the various steps in the tissue reconfiguring procedure by cable assembly 716. End effector 718 includes first and second jaw members 720, 722 which engage tissue 704. Cable assembly 716 includes first and second cable pairs 724a, 724b, and 726a, 726b for moving jaws 720, 722 relatively toward and away from one another, respectively, in a first plane, and a third cable 728 for moving end effector 718 relative to shaft 710 in a second plane generally transverse to, and preferably perpendicular to, the first plane, as described further below. During insertion into the stomach, end effector 718 is aligned with shaft 710 (as shown in Fig. 40A). Once positioned in the stomach, cable 728 is actuated to articulate end effector 718 out of alignment with shaft 710 (as shown in Fig. 38).

Cable assembly 716 includes a spring beam 784, formed from, e.g., stainless steel, extending into shaft 710. End effector 718 is attached to beam 784 at a distal end 785 of beam 784. Beam 784, in its rest state, is biased toward a straight alignment. Pulling cable 728 bends beam 784. When cable 728 is released, beam 784 returns toward the straight alignment.

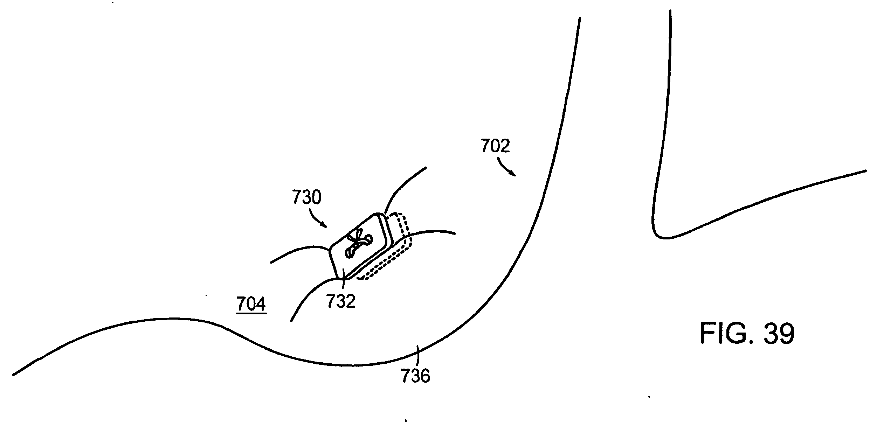

Referring also to Fig. 39, mounted to first jaw 720 is a first part 732 of a tissue securement member, e.g., a fixation device 730, and mounted to second jaw 722 is a second part 734 of tissue fixation device 730. As described further below, after jaws 720, 722 engage tissue 704 and manipulate the tissue in a wrapping action to create a bulge 736 in, e.g., the lesser curvature of the stomach, tissue fixation device 730 is deployed to secure the engaged tissue together. Cable assembly 7.16 includes a fourth cable 737 for deploying fixation device 730, as described further below.

End effector 718 further includes a tube 738 and a third tissue engaging member, e.g., a coil 740, received within tube 738, for purposes described below. Coil 740 is housed within an overtube 742, and coil 740 and overtube 742 can be moved axially proximally and distally relative to jaws 720, 722, along the axis, A, of cable assembly 716. Coil 740 can be rotatably advanced into tissue.

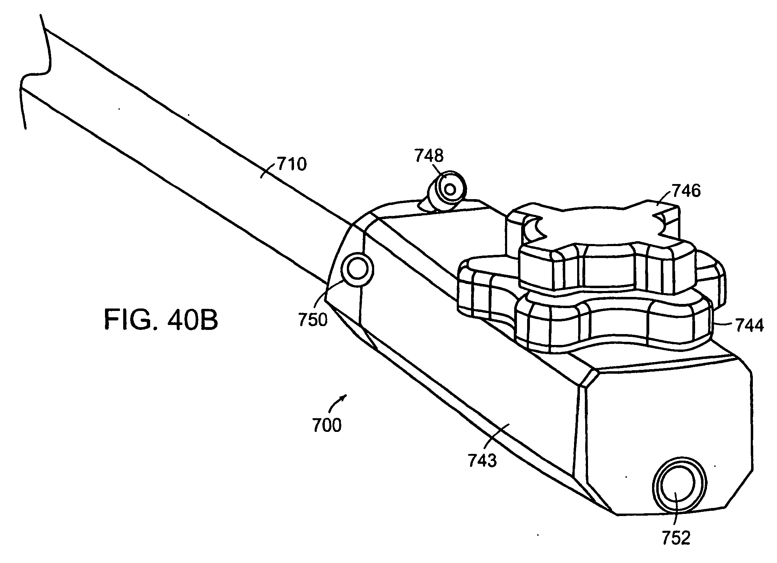

Referring to Fig. 40A, instrument 700 has, at its proximal end 745, a handle 743 with a control knob 744 for controlling cables 724a, 724b, 726a, 726b to close and open jaws 720, 722, and a control knob 746 for controlling cable 728 to move end effector 718. Handle 743 includes a port 748 through which coil 740 and overtube 742 can be introduced into shaft lumen 714, and a pull-knob 750 for deploying tissue fixation device 730, as described below. As shown in Fig. 40B, handle 743 defines a channel 752 through which endoscope 715 is introduced into shaft lumen 714.

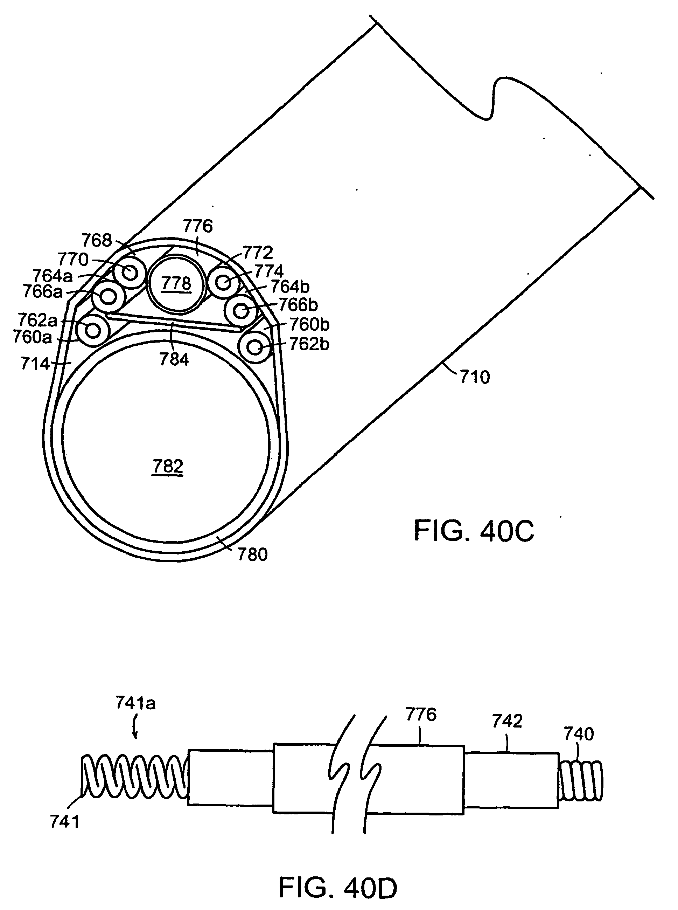

Referring to Figs. 38 and 40C, which shows the working channels in shaft 710 for receiving the various cables, overtube 742 and endoscope 715, within lumen 714 of shaft 710 are cable housings 760a, 760b defining channels 762a, 762b in which cables 724a, 724b for closing jaws 720, 722 are received, and cable housings 764a, 764b defining channels 766a, 766b in which cables 726a, 726b for opening jaws 720, 722 are received. Within lumen 714 are also a cable housing 768 defining a channel 770 in which cable 728 for bending end effector 718 is received, and a cable housing 772 defining a channel 774 in which cable 737 for deploying fixation device 730 is received. Coil 740 and overtube 742 are received in a channel 778 defined in a coil housing 776 in lumen 714. Housing 776 extends from port 748 to tube 738. As shown in Fig. 40D, coil 740 has a tissue penetrating tip 741 and a distal section 740a having a looser wound coil than the remainder of coil 740. Endoscope 715 is received in a channel 782 defined in an endoscope housing 780 in lumen 715.

Spring beam 784 is located generally between cable housing 776 and endoscope housing 780, and extends about 102mm (4 inches) into shaft 710 from the distal end of the shaft where beam 784 is mounted to shaft 710 by, e.g., silicone adhesive/sealant. The various cable housings and spring beam 784 do not move relative to shaft 710 and handle 743. It is the movement of the cables within the cable housings that actuate end effector 718. Shaft 710 is preferably formed from, e.g., heat-shrink tubing.

Referring again to Fig. 40A, end effector 718 has a length, L1, of about 50.8mm (2 inches), cable assembly 716 extends axially by a length, L2, of about 63.5mm (2.5 inches) from shaft 710, shaft 710 has a length, L3, of about 597mm (23.5 inches), and handle 743 has a length, L4, of about 127mm (5 inches). Cable assembly 716, spring beam 784, and shaft 710 have the necessary flexibility to permit transoral placement of instrument 700 into the stomach. The length, L1, of relatively rigid end effector 718 is minimized to ensure the necessary flexibility of instrument 700 is maintained. The distance that cable assembly 716 extends axially from shaft 710 is selected to cantilever beam 784 permitting the desired bending of end effector 718 relative to shaft 710 to position jaws 720, 722 against the inner surface of the stomach in the vicinity of the GEJ.

Distal end effector 718 is sized to fit through a 12-16 mm diameter channel (corresponding to the diameter of the esophagus) and shaft 710 has an outer diameter of about 12 to 16 mm to enable transoral passage of instrument 700 into the stomach. Scope channel 782 has a diameter of either about 8 mm or 10 mm. An 8 mm diameter scope channel allows passage of 7.9 mm pediatric gastroscope, and a 10 mm diameter scope channel allows passage of a 9.8 mm adult gastroscope. Channel 778 has a diameter of about 2-3 mm for receiving cable 742.

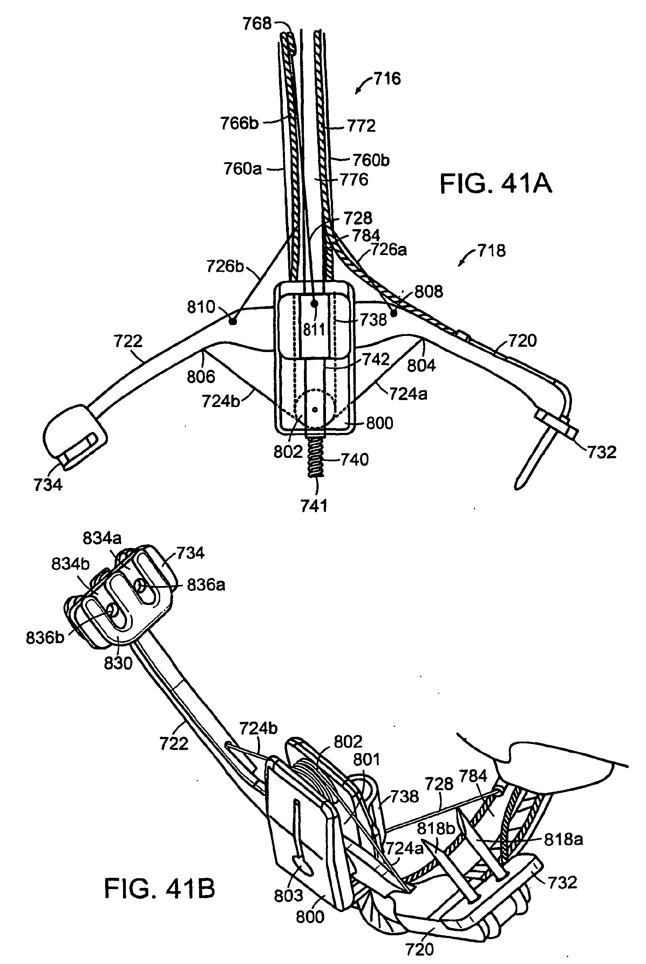

Distal end effector 718 is shown in more detail in Figs. 41A and 41B. End effector 718 includes a central mount 800 defining a slot 801. Spanning slot 801 and supported by mount 800 is a pin 803 to which 720, 722 are pivotally mounted. Central mount 800 also houses two pulleys 802 over which cables 724a, 724b are respectively passed for closing jaws 720, 722. Cables 724a, 724b terminate at points 804, 806 on jaws 720, 722, respectively. Cables 726a, 726b for opening jaws 720, 722 terminate at points 808, 810 on jaws 720, 722, respectively, proximal of points 804, 806. Tube 738 of end effector 718 for receiving coil 740 and overtube 742 is attached to mount 800, and cable 728 for bending end effector 718 terminates at point 811 on tube 738.

Pulling cables 724a, 724b proximally moves jaws 720, 722 toward one another generally in a first plane (in the plane of the paper in Fig. 41A). Pulling cables 726a, 726b proximally moves jaws 720, 722 away from one another generally in the first plane. Pulling cable 728 proximally bends beam 784 moving end effector 718 in a second plane (out of the plane of the paper in Fig. 41A) generally perpendicular to the first plane.

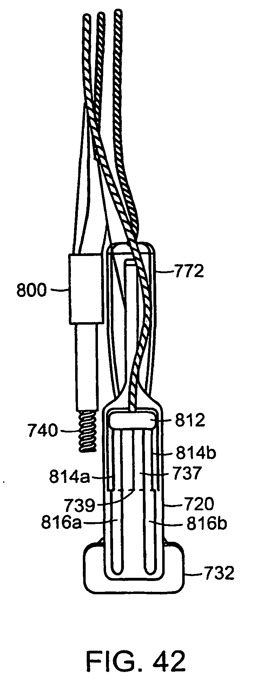

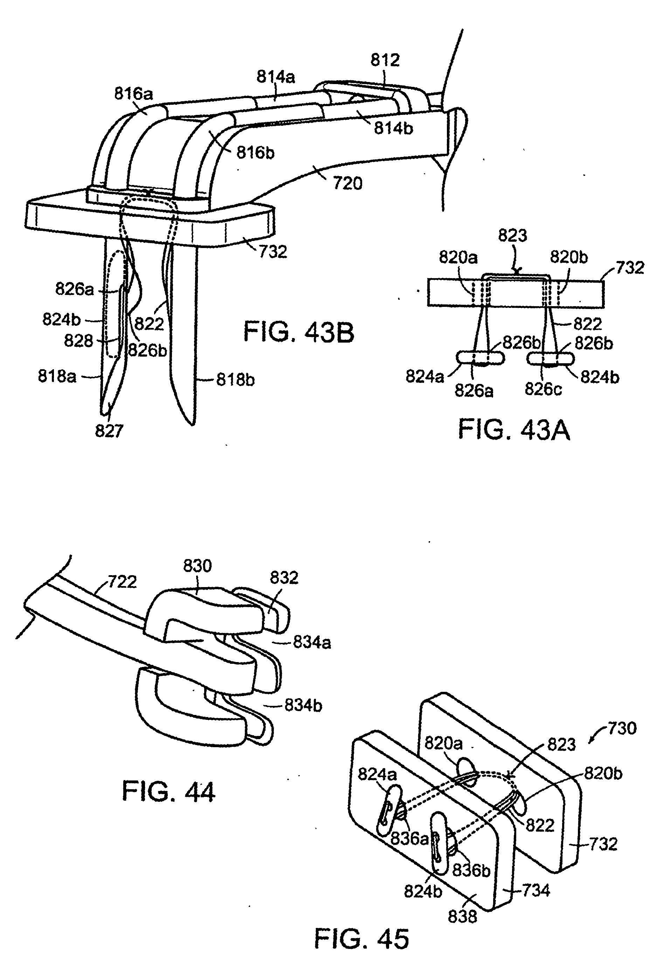

Referring also to Fig. 42, jaw 720 includes two guide tubes 816a, 816b and a slider 812 including two push rods 814a, 814b guided within tubes 816a, 816b, respectively. Slider 812 is mounted to jaw 720 to slide relative to jaw 720. Tubes 816a, 816b curve about jaw 720 to terminate in tissue penetrating tips 818a, 818b (Fig. 43B), respectively. Push rods 814a, 814b can be formed from molded plastic such as polyethylene or polypropylene or as a braided stainless steel cable to provide the flexibility to follow the curve of tubes 816a, 816b. Cable housing 772 is attached to slider 812 and cable 737 terminates at a fixed point 739 on jaw 720. Actuation of cable 737 pushes slider 812 distally, as described below.

First part 732 of tissue fixation device 730 is shown in more detail in Figs. 43A and 43B. First part 732 of tissue fixation device 730 defines through holes 820a, 820b (Fig. 43A), and part 732 is loaded onto jaw 720 with tips 818a, 818b received in through holes 820a, 820b, respectively. Connected to part 732 with a suture 822 are two securing elements, e.g., bars 824a, 824b. Each bar 824a, 824b defines two through holes 826a, 826b. Suture 822 is threaded through holes 826a, 826b of the bars and through holes 820a, 820b of part 732, and is tied together forming a knot 823 to secure bars 824a, 824b to part 732. Tubes 818a, 818b each define a channel 827 for receiving one of bars 824a, 824b, and a slot 828 communicating with channel 827 for receiving suture 822 therethrough.

Referring particularly to Figs. 41B and 44, jaw 722 has a distal member 830 defining a slot 832 for receiving second part 734 of fixation device 730, and slots 834a, 834b for receiving tissue penetrating tips 818a, 818b. Second part 734 of fixation device 730 defines through holes 836a, 836b for receiving tips 818a, 818b. When jaws 720, 722 are closed, tips 818a, 818b pass through slots 834a, 834b and holes 836a, 836b. Actuation of fixation device deployment cable 737 after closing jaws 720, 722 pushes slider 812 and push rods 814a, 814b distally, advancing bars 824a, 824b out of tissue penetrating tips 818a, 818b, and locating bars 824a, 824b on the far side 838 of second part 734 of fixation device 730, as shown in Fig. 45.

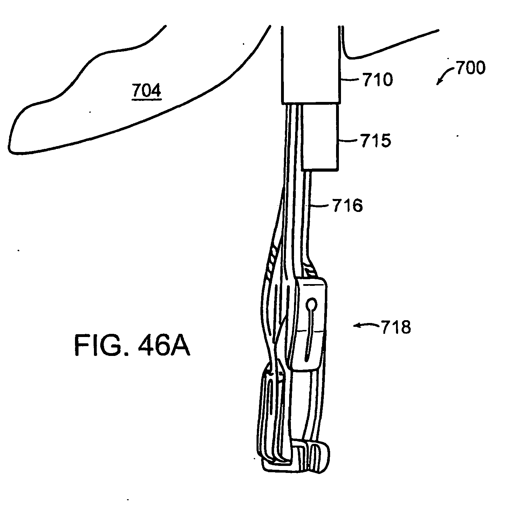

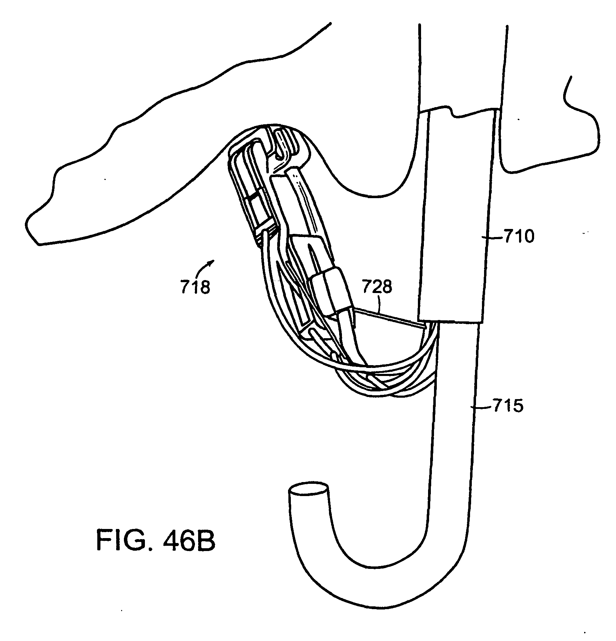

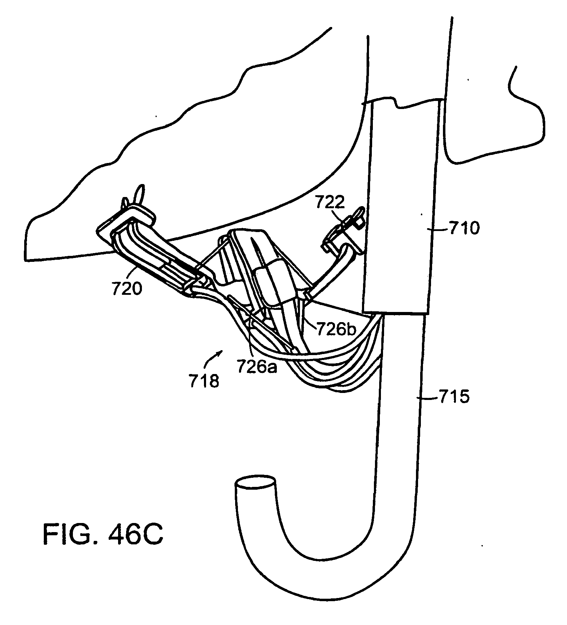

Referring to Figs. 46A-46F, in use, under endoscopic guidance, the physician advances instrument 700 transorally to position end effector 718 in the stomach. During advancement into the stomach, end effector 718 is generally aligned along the axis of shaft 710, as shown in Fig. 46A. The physician then turns control knob 746 to pull cable 728 proximally, thereby bending beam 784 moving end effector 718 out of alignment with shaft 710 to the position shown in Fig. 46B. By then turning control knob 744 to pull cables 726a, 726b, jaws 720, 722 are pivoted about pins 803 to the open position shown in Fig. 46C.

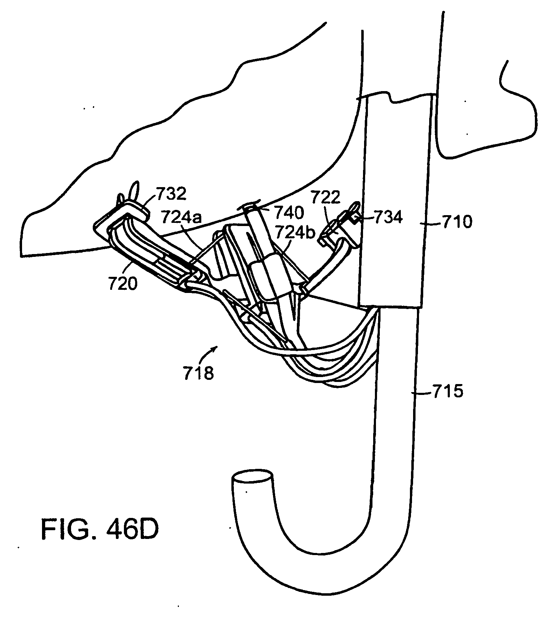

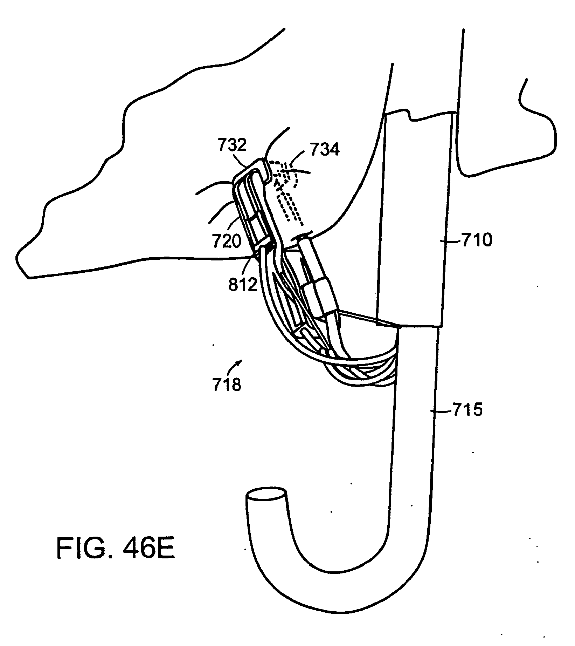

The physician then advances coil 740 and overtube 742 by pushing the coil and overtube distally in channel 778 advancing coil 740 and overtube 742 out of tube 738 and into contact with stomach tissue, preferably stomach tissue beyond the gastroesophageal junction, as shown in Fig. 38. With overtube 742 pressing against the tissue to stabilize the tissue, the physician rotates coil 740 while applying slight distal pressure to advance the coil into the tissue, as shown in Fig. 46D. Coil 740 and overtube 742 are then pulled proximally to pull tissue between jaws 720, 722. Jaws 720, 722 are then closed by turning control knob 744 to pull cables 724a, 724b proximally, as shown in Fig. 46E. The turning of the control knob can also be the action that pulls coil 740 and overtube 742 proximally, ensuring that coil 740 and overtube 742 are positioned out of the way of the closing of the jaws. A lockout can be incorporated to prevent the jaws from closing if coil 740 and overtube 742 are not in their proximal position.

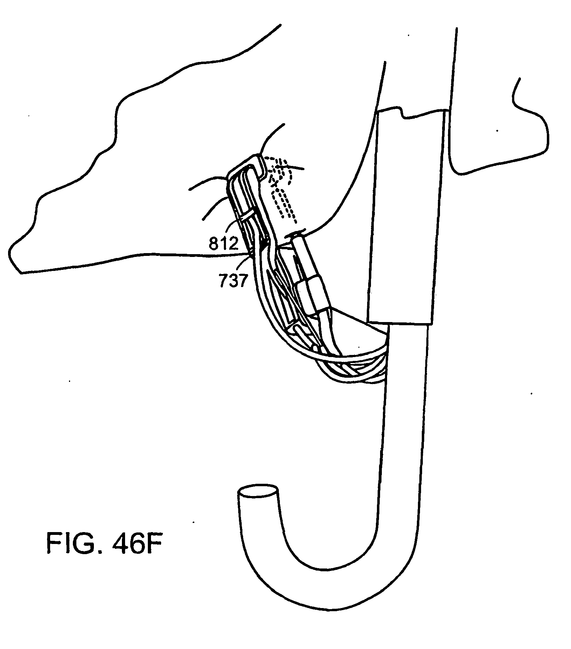

The closing of the jaws places parts 732, 734 of fixation device 730 in contact with two tissue sections, e.g., against two spaced tissue surfaces in the stomach, and causes tissue penetrating tips 818a, 818b to penetrate through the tissue and into holes 836a, 836b in second part 734 of fixation device 730. To deploy fixation device 730, the physician pulls cable 737 proximally removing slack from cable 737. Because cable housing 772 is of fixed length and is non-movably attached to the handle, removing slack from cable 737 causes cable housing 772 to move distally, advancing slider 812 to push t-bars 824a, 824b out of tissue penetrating tips 818a, 818b, as shown in Fig. 46F.

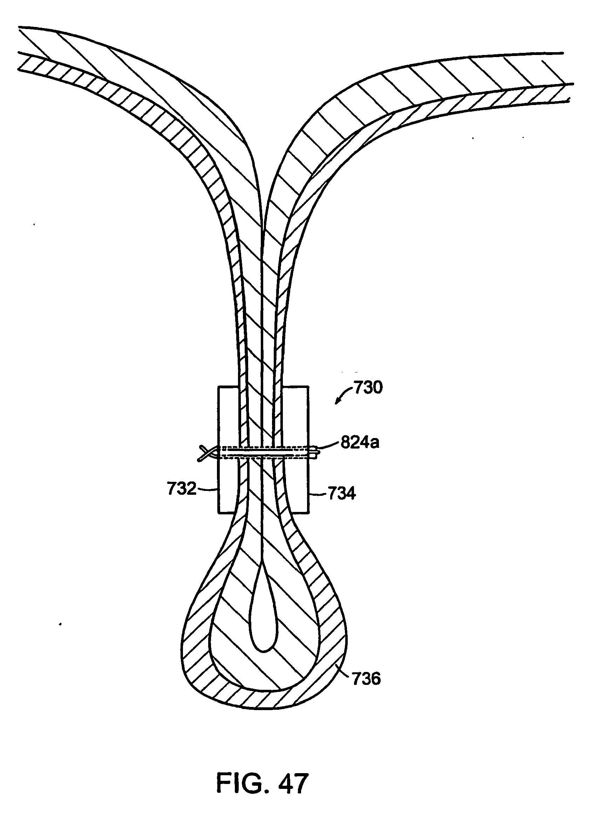

The physician then opens the jaws, disengages jaw 722 from second part 734, returns the distal end effector to its original position generally aligned with shaft 710, closes the jaws and removes instrument 700. Fig. 47 shows a cross-section of the tissue with fixation device 730 in place securing bulge 736.

Other embodiments of the inventive apparatus are within the scope of the following claims.

For example, rather than a coil 740, alternative tissue penetrating or grasping elements such as a T-bar suture or two small grasping jaws can be employed. Instrument 700 can be used without the third tissue engaging member.

The instrument and fixation device of Fig. 38 can be used to repair a hiatus hernia, as described above.

## Claims

1. Apparatus comprising:
an elongated member (710) configured for transoral placement into a stomach, and
a distal end effector (718) including first and second members (720,722) configured to engage stomach tissue (704), the first and second members being movable relatively toward one another generally in a first plane, **characterized by** the distal end effector being movable relative to the elongated member in a second plane generally transverse to the first plane, such that the distal end effector is bent out of alignment with the elongated member.

2. The apparatus of claim 1 for use in a method of advancing the apparatus transorally into the stomach and moving the distal effector relative to the elongated member in the second plane to position the first and second members for engagement with the tissue.

3. The apparatus of claim 1 or 2 wherein the first and second members (720,722) are configured to engage stomach tissue (704) beyond a gastroesophageal junction (702).

4. The apparatus of any one of the preceding claims, wherein the distal end effector (718) further includes a third member (740) configured to engage stomach tissue, the third member being movable in a distal direction relative to the first and second members (720,722).

5. The apparatus of claim 4, wherein the third member (740) includes a tissue engaging portion preferably comprising a coil having a tissue penetrating tip (741).

6. The apparatus of any one of the preceding claims further including a tissue securement member for coupling to at least one of the first and second members (720,722) for securing together tissue engaged thereby.

7. The apparatus of claim 6 wherein the tissue securement member comprises a first part (732) for coupling to the first member (720) for engagement with a first tissue section,
a second part (734) for coupling to the second member (722) for engagement with a second tissue section to be secured to the first tissue section,
a suture (822) attached to the first part, and
a securing element (824a) attached to the suture and configured for engagement with the second part when the first and second members are moved relatively toward one another to engage the first and second tissue sections, thereby to secure the second part to the first part.

8. The apparatus of claim 7 wherein the securing element (824a) is configured for deployment from the first member (720).

9. The apparatus of claim 7 wherein the first member (720) includes a deploying element (737) for deploying the securing element (824a) from the first member.

10. The apparatus of any one of the preceding claims wherein the second plane is generally perpendicular to the first plane.

11. The apparatus of any one of the preceding claims wherein the distal end effector (718) is configured for movement between a first position generally aligned with the elongated member (710) and a second position in which the distal end effector has moved in the second plane out of alignment with the elongated member.

12. The apparatus of claim 11 further including an end effector cable (728) actuatable from a proximal end of the apparatus and coupled to the distal end effector (718) for moving the distal end effector in the second plane.

13. The apparatus of any one of the preceding claims further including a member cable (716) actuatable from a proximal end of the apparatus and coupled to the distal end effector (718) for moving the first and second members (720,722) generally in the first plane.

14. The apparatus of any one of the preceding claims wherein the first member (720) includes a tissue piercing element (818a).

15. The apparatus of claim 14 wherein the first member includes a second tissue piercing element (818b).

16. The apparatus of claim 14 or 15 wherein either or both tissue piercing elements (818a,818b) define a channel (827) for receiving a securing element (824a,824b).

17. The apparatus of any one of the preceding claims wherein the elongated member (710) defines a channel (714) for receiving an endoscope (715).

18. The apparatus of any one of the preceding claims further comprising means for endoscope visualisation (715).

19. The apparatus of claim 1 wherein the first member (720) includes first and second tissue piercing elements (818a,818b) each defining a channel (827) and the apparatus further comprises a third member (740) including a tissue engaging portion (741) for engaging stomach tissue, the third member being movable in a distal direction relative to the first and second members, and
a tissue securement member including a first part (732) for coupling to the first member for engagement with a first tissue section, a second part (734) for coupling to the second member (722) for engagement with a second tissue section to be secured to the first tissue section, a suture (822) attached to the first part, and first and second securing elements (824a, 824b) attached to the suture and deployable through the channels of the first and second tissue piercing elements for engagement with the second part when the first and second members move relatively toward one another to engage the first and second tissue sections, thereby to secure the second part to the first part.

20. The apparatus of claim 12 further including a spring beam (784) extending into the elongated member (710) and being biased toward a straight alignment, wherein the distal end effector (718) is attached to the beam (784).

21. The apparatus of claim 20 wherein the end effector cable (728) is configured to bend the beam (784) when pulled.

22. The apparatus of claim 13 wherein the member cable (716) is configured to move the first and second member (720, 722) toward or away from each other in the first plane.

23. The apparatus of claim 22 further comprising a control knob (744) at a proximal end of the apparatus for controlling the member cable (716).

24. The apparatus of claim 12 further comprising a control knob (746) at a proximal end of the apparatus for controlling the end effector cable (728).

25. The apparatus of claim 6 further comprising a tissue securement cable (737) actuatable form a proximal end of the apparatus and couple to the disal end effector (718) for deploying the tissue securement member.

26. The apparatus of claim 1, wherein the second member, (722) being moveable in a distal direction, is configured to engage stomach tissue.

27. The apparatus of claim 26, wherein the second member (722) includes a tissue engaging tip.

28. The apparatus of claim 27, wherein the tissue engaging tip comprises one of a corkscrew-type element, grasping forceps, cup forceps, fenestrated forceps or suction device.

29. An apparatus according to claim 1, wherein the first and second members (720, 722) are configured to manipulate stomach tissue into a reconfigured tissue position and the distal end effector (718) is configured to secure the stomach tissue in the reconfigured tissue position; and wherein the apparatus further comprises endoscopic visualization means (715) for visualization of the reconfigured tissue position within the stomach.

30. The apparatus of claim 29, wherein the elongated member (710) includes at least one tissue securing element (730) for securing the stomach tissue in the reconfigured tissue position.

31. The apparatus of claim 29 or 30, wherein the tissue securing element (730) comprises one of a biocompatible staple, a biocompatible clip, tack, rivet, two-part fastener, helical fastener, T-bar suture, or suture.

32. The apparatus of claim 30 or 31, wherein the distal end effector (718) is configured to cooperate with the tissue securing element (730) to secure the stomach tissue in the reconfigured position.

33. The apparatus of any of claims 29-32, wherein the first and second members (720, 722) are configured to engage stomach tissue beyond a gastroesophageal junction.

34. The apparatus of any of claims 29-33, wherein the first and second members (720, 722) further comprise a tissue engaging tip.

35. The apparatus of claim 34, wherein the tissue engaging tip comprises one of a corkscrew-type element, grasping forceps, cup forceps, fenestrated forceps or a suction device.

36. The apparatus of any of claims 29-35, wherein the distal end effector (718) and first and second members (720 722) are movable with respect to each other.

37. The apparatus of claims 29-36, wherein the distal end effector further comprises a suction device configured to engage stomach tissue.

38. The apparatus of claims 29-37, wherein the endoscopic visualization means (715) is manipulable in to a j-shape.

## Patentansprüche

1. Vorrichtung, die Folgendes umfasst:
ein längliches Element (710), das für eine transorale Platzierung in einem Magen konfiguriert ist, und
einen distalen Endeffektor (718) mit einem ersten und einem zweiten Element (720, 722), die so konfiguriert sind, dass sie in Magengewebe (704) eingreifen, wobei das erste und das zweite Element im Allgemeinen in einer ersten Ebene relativ zueinander bewegbar sind, **dadurch gekennzeichnet, dass** der distale Endeffektor relativ zu dem länglichen Element in einer zweiten Ebene allgemein quer zur ersten Ebene bewegbar ist, so dass der distale Endeffektor aus der Ausrichtung auf das längliche Element weg gekrümmt wird.

2. Vorrichtung nach Anspruch 1 zur Verwendung in einem Verfahren zum Vorwärtsbewegen der Vorrichtung transoral in den Magen und Bewegen des distalen Effektors relativ zu dem länglichen Element in der zweiten Ebene, um das erste und das zweite Element für einen Eingriff in das Gewebe zu positionieren.

3. Vorrichtung nach Anspruch 1 oder 2, wobei das erste und das zweite Element (720, 722) so konfiguriert sind, dass sie in Magengewebe (704) jenseits einer gastroösophagealen Verbindung (702) eingreifen.

4. Vorrichtung nach einem der vorherigen Ansprüche, wobei der distale Endeffektor (718) ferner ein drittes Element (740) beinhaltet, das so konfiguriert ist, dass es in Magengewebe eingreift, wobei das dritte Element in einer distalen Richtung relativ zum ersten und zum zweiten Element (720, 722) bewegbar ist.

5. Vorrichtung nach Anspruch 4, wobei das dritte Element (740) einen in Gewebe eingreifenden Abschnitt beinhaltet, der vorzugsweise eine Spule mit einer in Gewebe eindringenden Spitze (741) umfasst.

6. Vorrichtung nach einem der vorherigen Ansprüche, die ferner ein Gewebebefestigungselement zur Kopplung an das erste und/oder das zweite Element (720, 722) umfasst, um von diesen arretiertes Gewebe aneinander zu befestigen.

7. Vorrichtung nach Anspruch 6, wobei das Gewebebefestigungselement Folgendes umfasst: ein erstes Teil (732) zum Koppeln an das erste Element (720) für einen Eingriff in eine erste Gewebesektion,
ein zweites Teil (734) zum Koppeln an das zweite Element (722) für einen Eingriff in eine zweite Gewebesektion, die an der ersten Gewebesektion befestigt werden soll,
ein am ersten Teil befestigtes Nahtmaterial (822) und
ein Befestigungsteil (824a), das am Nahtmaterial angebracht und für einen Eingriff in das zweite Teil konfiguriert ist, wenn das erste und das zweite Element relativ zueinander bewegt werden, um in die erste und die zweite Gewebesektion einzugreifen, um so das zweite Teil am ersten Teil zu befestigen.

8. Vorrichtung nach Anspruch 7, wobei das Befestigungsteil (824a) für ein Ansetzen vom ersten Element (720) konfiguriert ist.

9. Vorrichtung nach Anspruch 7, wobei das erste Element (720) ein Ansatzteil (737) zum Ansetzen des Befestigungsteils (824a) vom ersten Element beinhaltet.

10. Vorrichtung nach einem der vorherigen Ansprüche, wobei die zweite Ebene allgemein lotrecht zur ersten Ebene ist.

11. Vorrichtung nach einem der vorherigen Ansprüche, wobei der distale Endeffektor (718) für eine Bewegung zwischen einer ersten Position, die allgemein auf das längliche Element (710) ausgerichtet ist, und einer zweiten Position konfiguriert ist, in der sich der distale Endeffektor in der zweiten Ebene aus einer Ausrichtung auf das längliche Element bewegt hat.

12. Vorrichtung nach Anspruch 11, die ferner ein Endeffektorkabel (728) beinhaltet, das von einem proximalen Ende der Vorrichtung zu betätigen ist und mit dem distalen Endeffektor (718) zum Bewegen des distalen Endeffektors in der zweiten Ebene gekoppelt ist.

13. Vorrichtung nach einem der vorherigen Ansprüche, die ferner ein Elementkabel (716) beinhaltet, das von einem proximalen Ende der Vorrichtung zu betätigen ist und mit dem distalen Endeffektor (718) zum Bewegen des ersten und des zweiten Elements (720, 722) allgemein in der ersten Ebene gekoppelt ist.

14. Vorrichtung nach einem der vorherigen Ansprüche, wobei das erste Element (720) ein gewebedurchstoßendes Teil (818a) beinhaltet.

15. Vorrichtung nach Anspruch 14, wobei das erste Element ein zweites gewebedurchstoßendes Teil (818b) beinhaltet.

16. Vorrichtung nach Anspruch 14 oder 15, wobei ein oder beide gewebedurchstoßende Elemente (818a, 818b) einen Kanal (827) zur Aufnahme eines Befestigungsteils (824a, 824b) definieren.

17. Vorrichtung nach einem der vorherigen Ansprüche, wobei das längliche Element (710) einen Kanal (714) zur Aufnahme eines Endoskops (715) definiert.

18. Vorrichtung nach einem der vorherigen Ansprüche, die ferner ein Mittel zur Endoskopvisualisierung (715) umfasst.

19. Vorrichtung nach Anspruch 1, wobei das erste Element (720) ein erstes und ein zweites gewebedurchstoßendes Teil (818a, 818b) beinhaltet, die jeweils einen Kanal (827) definieren, und die Vorrichtung ferner ein drittes Element (740) umfasst, das einen in Gewebe eingreifenden Abschnitt (741) für einen Eingriff in Magengewebe beinhaltet, wobei das dritte Element in einer distalen Richtung relativ zum ersten und zweiten Element bewegbar ist, sowie
ein Gewebebefestigungselement mit einem ersten Teil (732) zum Koppeln an das erste Element für einen Eingriff in eine erste Gewebesektion, einem zweiten Teil (734) zum Koppeln an das zweite Element (722) für einen Eingriff in eine zweite Gewebesektion, die an der ersten Gewebesektion befestigt werden soll, ein Nahtmaterial (822), das am ersten Teil angebracht ist, und ein erstes und zweites Befestigungsteil (824a, 824b), die an dem Nahtmaterial angebracht und durch die Kanäle des ersten und zweiten gewebedurchstoßenden Teils für einen Eingriff in das zweite Teil ansetzbar sind, wenn sich das erste und das zweite Element relativ zueinander bewegen, um in die erste und zweite Gewebesektion einzugreifen, um folglich das zweite Teil am ersten Teil zu befestigen.

20. Vorrichtung nach Anspruch 12, die ferner einen Federbalken (784) beinhaltet, der sich in das längliche Element (710) erstreckt und in Richtung auf eine gerade Ausrichtung vorgespannt ist, wobei der distale Endeffektor (718) an dem Federbalken (784) befestigt ist.

21. Vorrichtung nach Anspruch 20, wobei das Endeffektorkabel (728) so konfiguriert ist, dass sich der Federbalken (784) krümmt, wenn daran gezogen wird.

22. Vorrichtung nach Anspruch 13, wobei das Elementkabel (716) so konfiguriert ist, dass das erste und das zweite Element (720, 722) aufeinander zu oder voneinander weg in der ersten Ebene bewegt werden.

23. Vorrichtung nach Anspruch 22, die ferner einen Bedienknopf (744) an einem proximalen Ende der Vorrichtung zum Steuern des Elementkabels (716) umfasst.

24. Vorrichtung nach Anspruch 12, die ferner einen Bedienknopf (746) an einem proximalen Ende der Vorrichtung zum Steuern des Endeffektorkabels (728) umfasst.

25. Vorrichtung nach Anspruch 6, die ferner ein Gewebebefestigungskabel (737) umfasst, das von einem proximalen Ende der Vorrichtung zu betätigen ist und an den distalen Endeffektor (718) zum Ansetzen des Gewebebefestigungselements gekoppelt ist.

26. Vorrichtung nach Anspruch 1, wobei das zweite Element (722), das in einer distalen Richtung bewegbar ist, so konfiguriert ist, dass es in Magengewebe eingreift.

27. Vorrichtung nach Anspruch 26, wobei das zweite Element (722) eine in Gewebe eingreifende Spitze beinhaltet.

28. Vorrichtung nach Anspruch 27, wobei die in Gewebe eingreifende Spitze ein korkenzieherartiges Teil, Greifzangen, Becherzangen, gefensterte Zangen oder eine Saugvorrichtung umfasst.

29. Vorrichtung nach Anspruch 1, wobei das erste und das zweite Element (720, 722) so konfiguriert sind, dass Magengewebe in eine rekonfigurierte Gewebeposition manipuliert wird und der distale Endeffektor (718) so konfiguriert ist, dass Magengewebe in der rekonfigurierten Gewebeposition befestigt wird, und wobei die Vorrichtung ferner ein endoskopisches Visualisierungsmittel (715) zur Visualisierung der rekonfigurierten Gewebeposition im Magen umfasst.

30. Vorrichtung nach Anspruch 29, wobei das längliche Element (710) wenigstens ein Gewebebefestigungsteil (730) zum Befestigen von Magengewebe in der rekonfigurierten Gewebeposition beinhaltet.

31. Vorrichtung nach Anspruch 29 oder 30, wobei das Gewebebefestigungsteil (730) eine biokompatible Heftklammer, eine biokompatible Klemme, einen Stift, einen Niet, ein zweiteiliges Befestigungsmittel, ein schraubenförmiges Befestigungsmittel, eine "T-Bar"-Naht oder Nahtmaterial umfasst.

32. Vorrichtung nach Anspruch 30 oder 31, wobei der distale Endeffektor (718) so konfiguriert ist, dass er mit dem Gewebebefestigungsteil (730) zusammenwirkt, um das Magengewebe in der rekonfigurierten Position zu befestigen.

33. Vorrichtung nach einem der Ansprüche 29-32, wobei das erste und das zweite Element (720, 722) so konfiguriert sind, das sie in Magengewebe jenseits einer gastroösophagealen Verbindung eingreifen.

34. Vorrichtung nach einem der Ansprüche 29-33, wobei das erste und das zweite Element (720, 722) ferner eine in Gewebe eingreifende Spitze umfassen.

35. Vorrichtung nach Anspruch 34, wobei die in Gewebe eingreifende Spitze ein korkenzieherartiges Teil, Greifzangen, Becherzangen, gefensterte Zangen oder eine Saugvorrichtung umfasst.

36. Vorrichtung nach einem der Ansprüche 29-35, wobei der distale Endeffektor (718) und das erste und das zweite Element (720, 722) mit Bezug aufeinander bewegbar sind.

37. Vorrichtung nach Anspruch 29-36, wobei der distale Endeffektor ferner eine Saugvorrichtung umfasst, die so konfiguriert ist, dass sie in Magengewebe eingreift.

38. Vorrichtung nach Anspruch 29-37, wobei das endoskopische Visualisierungsmittel (715) in eine j-Gestalt manipulierbar ist.

## Revendications

1. Dispositif comprenant :
un membre allongé (710) configuré pour permettre la mise en place, par voie transorale, dans un estomac et
un organe terminal (718) distal qui comprend un premier et un second membres (720, 722) configurés pour venir en contact avec le tissu gastrique (704), les premier et second membres pouvant subir un déplacement relatif l'un vers l'autre généralement dans un premier plan, **caractérisé en ce que** l'organe terminal distal est déplaçable par rapport au membre allongé dans un second plan généralement transversal au premier plan de telle sorte que l'organe terminal distal est fléchi hors d'alignement avec le membre allongé.

2. Dispositif de la revendication 1, qui est conçu pour être utilisé dans une méthode qui fait intervenir une introduction du dispositif dans l'estomac par voie transorale et un déplacement dans le second plan de l'organe terminal distal par rapport au membre allongé de façon à ce que les premier et second membres soient positionnés pour venir en contact avec le tissu.

3. Dispositif de la revendication 1 ou 2, où les premier et second membres (720, 722) sont configurés de façon à venir en contact avec le tissu gastrique (704) en aval de la jonction gastro-oesophagienne (702).

4. Dispositif de l'une quelconque des revendications précédentes, où l'organe terminal distal (718) comprend également un troisième membre (740) configuré pour venir en contact avec le tissu gastrique, le troisième membre étant déplaçable en une direction distale par rapport aux premier et second membres (720, 722).

5. Dispositif de la revendication 4, où le troisième membre (740) comprend une portion qui vient en contact avec le tissu et qui comprend préférablement un serpentin doté à son extrémité d'une pointe de pénétration tissulaire (741).

6. Dispositif de l'une quelconque des revendications précédentes, qui comprend également un membre d'ancrage au tissu qui peut être couplé à un au moins des premier et second membres (720, 722) pour l'arrimer au tissu avec lequel il est en contact.

7. Dispositif de la revendication 6, où le membre d'ancrage au tissu comprend une première partie (732) qui peut être couplée au premier membre (720) pour le mettre en contact avec une première section de tissu,
une seconde partie (734) qui peut être couplée au second membre (722) pour le mettre en contact avec une seconde section de tissu à arrimer à la première section de tissu,
une suture (822) rattachée à la première partie et
un élément de fixation (824a) rattaché à la suture et configuré de façon à venir en contact avec la seconde partie quand les premier et second membres sont soumis à un déplacement relatif l'un vers l'autre afin de venir en contact avec les première et seconde sections de tissu, produisant ainsi un arrimage de la seconde partie à la première partie.

8. Dispositif de la revendication 7, où l'élément de fixation (824a) est configuré de façon à pouvoir être déployé à partir du premier membre (720).

9. Dispositif de la revendication 7, où le premier membre (720) comprend un élément de déploiement (737) pour déployer l'élément de fixation (824a) à partir du premier membre.

10. Dispositif de l'une quelconque des revendications précédentes, où le second plan est généralement perpendiculaire au premier plan.

11. Dispositif de l'une quelconque des revendications précédentes, où l'organe terminal distal (718) est configuré de façon à pouvoir être déplacé entre une première position généralement en alignement avec le membre allongé (710) et une seconde position dans laquelle l'organe terminal distal est déplacé dans le second plan hors d'alignement avec le membre allongé.

12. Dispositif de la revendication 11, qui comprend également un filin de l'organe terminal (728) qui peut être actionné à partir d'une extrémité proximale du dispositif et qui est couplé à l'organe terminal distal (718) pour déplacer l'organe terminal distal dans le second plan.

13. Dispositif de l'une quelconque des revendications précédentes, qui comprend également un filin de membre (716) qui peut être actionné à partir d'une extrémité proximale du dispositif et être couplé à l'organe terminal distal (718) pour déplacer les premier et second membres (720, 722) généralement dans le premier plan.

14. Dispositif de l'une quelconque des revendications précédentes, où le premier membre (720) comprend un élément de perçage tissulaire (818a).

15. Dispositif de la revendication 14, où le premier membre comprend un second élément de perçage tissulaire (818b).

16. Dispositif de la revendication 14 ou 15, où l'un des éléments de perçage tissulaire ou les deux (818a, 818b) définissent une rainure (827) où un élément de fixation peut être inséré (824a, 824b).

17. Dispositif de l'une quelconque des revendications précédentes, où le membre allongé (710) définit une rainure (714) où un endoscope peut être inséré (715).

18. Dispositif de l'une quelconque des revendications précédentes, qui comprend également un moyen de visualisation endoscopique (715).

19. Dispositif de la revendication 1, où le premier membre (720) comprend un premier et un second éléments de perçage tissulaire (818a, 818b) qui définissent chacun une rainure (827), le dispositif comprenant également un troisième membre (740) qui comporte une portion adaptée pour venir en contact avec le tissu (741) pour produire un contact avec le tissu gastrique, le troisième membre pouvant être déplacé en direction distale par rapport aux premier et second membres, et
un membre d'ancrage au tissu qui comporte une première partie (732) qui peut être couplée au premier membre pour le mettre en contact avec une première section de tissu, une seconde partie (734) qui peut être couplée au second membre (722) pour le mettre en contact avec une seconde section de tissu à arrimer à la première section de tissu, une suture (822) rattachée à la première partie et un premier et un second éléments de fixation (824a, 824b) rattachés à la suture et déployables par l'intermédiaire des rainures des premier et second éléments de perçage tissulaire de façon venir en contact avec la seconde partie quand les premier et second membres sont soumis à un déplacement relatif l'un vers l'autre afin de venir en contact avec les première et seconde sections de tissu, produisant ainsi un arrimage de la seconde partie à la première partie.

20. Dispositif de la revendication 12, qui comprend également une lamelle à ressort (784) qui se prolonge jusqu'à l'intérieur du membre allongé (710) en direction oblique pour produire un alignement direct, où l'organe terminal distal (718) est rattaché à la lamelle (784).

21. Dispositif de la revendication 20, où le filin de l'organe terminal 728) est configuré de façon à courber la lamelle (784) quand il est tiré.

22. Dispositif de la revendication 13, où le filin de membre (716) est configuré de façon à déplacer les premier et second membres (720, 722) l'un vers l'autre ou en direction opposée dans le premier plan.

23. Dispositif de la revendication 22, qui comprend également un bouton de commande (744) à l'extrémité proximale du dispositif pour contrôler le filin de membre (716).

24. Dispositif de la revendication 12, qui comprend également un bouton de commande (746) à une extrémité proximale du dispositif pour contrôler le filin de l'organe terminal (728).

25. Dispositif de la revendication 6, qui comprend également un filin d'arrimage du tissu (737) actionnable à partir d'une extrémité proximale du dispositif et dont le couplage à l'organe terminal distal (718) produit le déploiement du membre d'ancrage au tissu.

26. Dispositif de la revendication 1, où le second membre, (722), qui peut être déplacé en une direction distale, est configuré de façon à venir en contact avec le tissu gastrique.

27. Dispositif de la revendication 26, où le second membre (722) comprend un embout de mise en contact avec le tissu.

28. Dispositif de la revendication 27, où l'embout de mise en contact avec le tissu comprend l'un des composants suivants : élément du type en tire-bouchon, pince de préhension, pince à biopsie à large cuillère, pince à cuillère fenêtrée ou dispositif de succion.

29. Dispositif selon la revendication 1, où les premier et second membres (720, 722) sont configurés de façon à manipuler le tissu gastrique en une position tissulaire reconfigurée et où l'organe terminal distal (718) est configuré de façon à arrimer le tissu gastrique dans la position tissulaire reconfigurée ; et où le dispositif comprend également un moyen de visualisation endoscopique (715) permettant la visualisation de la position tissulaire reconfigurée dans l'estomac.

30. Dispositif de la revendication 29, où le membre allongé (710) comprend au moins un élément de fixation du tissu (730) permettant d'arrimer le tissu gastrique dans la position tissulaire reconfigurée.

31. Dispositif de la revendication 29 ou 30, où l'élément de fixation du tissu (730) comprend un des composants suivants : agrafe biocompatible, clip biocompatible, broquette, rivet, attache à deux éléments, attache hélicoïdale, suture en T ou suture.

32. Dispositif de la revendication 30 ou 31, où l'organe terminal distal (718) est configuré pour coopérer avec l'élément de fixation du tissu (730) de façon à arrimer le tissu gastrique en la position reconfigurée.

33. Dispositif de l'une quelconque des revendications 29-32, où les premier et second membres (720, 722) sont configurés de façon à venir en contact avec le tissu gastrique en aval de la jonction gastro-oesophagienne.

34. Dispositif de l'une quelconque des revendications 29-33, où les premier et second membres (720, 722) comprennent également un embout de mise en contact avec le tissu.

35. Dispositif de la revendication 34, où l'embout de mise en contact avec le tissu comprend l'un des composants suivants : élément du type en tire-bouchon, pince de préhension, pince à biopsie à large cuillère, pince à cuillère fenêtrée ou dispositif de succion.

36. Dispositif de l'une quelconque des revendications 29-35, où l'organe terminal distal (718) et les premier et second membres (720, 722) peuvent être déplacés les uns par rapport aux autres.

37. Dispositif des revendications 29-36, où l'organe terminal distal comprend également un dispositif de succion configuré pour venir en contact avec le tissu gastrique.

38. Dispositif des revendications 29-37, où le moyen de visualisation endoscopique (715) est manipulable en une forme en J.
